# EUROPEAN PATENT APPLICATION

(11) **EP 3 168 305 A1**
(43) Date of publication of application: **17.05.2017**
(21) Application number: 15818219.6
(22) Date of filing: 10.07.2015
(51) Int. Cl.: C12N 15/113, A61K 31/711, A61P 35/00

(54) **ANTISENSE ANTINEOPLASTIC AGENT**

(30) Priority: 10.07.2014 US 201462023067 P
(71) Applicant: Nakano, Kenji, Fukuoka-shi, Fukuoka 812-8581 (JP)
(72) Inventor: OBIKA, Satoshi, Suita-shi Osaka 565-0871 (JP); YAMAMOTO, Tsuyoshi, Suita-shi Osaka 565-0871 (JP)
(74) Representative: Vossius & Partner Patentanwälte Rechtsanwälte mbB
(86) International application number: PCT/JP2015/069947
(87) International publication number: WO 2016/006697

(57) **Abstract**

The present invention provides an oligonucleotide having a 13- to 17-nucleotide length, which comprises a part of the sequence of SEQ ID NO: 1 or 2 having at least 8-nucleotide length, and regulates expression of YB-1, as an artificial nucleic acid antisense anti-malignant tumor agent that can exhibit an effect against a wide range of malignant tumors in living bodies without any delivery device. The oligonucleotide preferably consists of the sequence of SEQ ID NO: 2, and has a bridged saccharide moiety type nucleoside structure, and phosphorothioate type internucleotide linkage.

SEQUENCE LISTING

<110>  Nakano, Kenji

<120>  ANTISENSE ANTICANCER AGENTS

<130>  151430H

<150>  U.S. Provisional Patent Application No. 62/023,067
<151>  2014-07-10

<160>  10

<170>  PatentIn version 3.5

<210>  1
<211>  15
<212>  DNA
<213>  Artificial Sequence

<220>
<223>  antisense oligonucleotide (ASO) #1

<400>  1
aggtgggaac cttcg                                                        15


<210>  2
<211>  15
<212>  DNA
<213>  Artificial Sequence

<220>
<223>  antisense oligonucleotide (ASO) #10

<400>  2
tctcctgcac cctgg                                                        15


<210>  3
<211>  15
<212>  DNA
<213>  Artificial Sequence

<220>
<223>  antisense oligonucleotide (ASO) control

<400>  3
catttcgaag tactc                                                        15


<210>  4
<211>  15
<212>  DNA
<213>  Artificial Sequence

<220>
<223>  antisense oligonucleotide (ASO) #N-7

<400>  4
cctgcaccct ggttg                                                        15

<210>  5
<211>  18
<212>  DNA
<213>  Artificial Sequence

<220>
<223>  antisense oligonucleotide (ASO) #N-14

<400>  5
tctcctgcac cctggttg                                                    18


<210>  6
<211>  18
<212>  DNA
<213>  Artificial Sequence

<220>
<223>  antisense oligonucleotide (ASO) #N-16

<400>  6
gttctcctgc accctggt                                                    18


<210>  7
<211>  18
<212>  DNA
<213>  Artificial Sequence

<220>
<223>  antisense oligonucleotide (ASO) #N-17

<400>  7
tgttctcctg caccctgg                                                    18


<210>  8
<211>  18
<212>  DNA
<213>  Artificial Sequence

<220>
<223>  antisense oligonucleotide (ASO) #N-18

<400>  8
ttgttctcct gcaccctg                                                    18


<210>  9
<211>  14
<212>  DNA
<213>  Artificial Sequence

<220>
<223>  antisense oligonucleotide (ASO) #N50

<400>  9
tgcatagata atgc                                                        14


<210>  10
<211>  14
<212>  DNA

<213>  Artificial Sequence

<220>
<223>  antisense oligonucleotide (ASO) #N25

<400>  10
tctcctgcac cctg                                                          14

## Description

### Technical Field

The present invention relates to an antisense anti-malignant tumor agent. The present invention is useful in the fields of drug, medical science, life science, and so forth.

### Background Art

The transcription factor Y-box binding protein 1 (YB-1) is specifically overexpressed in tumor cells and tumor vessels, and contributes to proliferation and therapeutic resistance of tumors. Patent document 1 discloses a method for detecting vascular endothelial cells of tumor neovascularity, which comprises measuring expression of YB-1, and is based on the finding that YB-1 is a biomarker specific to vascular endothelial cells of tumor neovascularity, and by suppressing expression of YB-1, tumor neovascularization can be suppressed, and as a result, proliferation of tumor can be inhibited. Patent document 2 discloses an oligonucleotide that can specifically hybridize to another oligonucleotide, especially a nucleic acid encoding human YB-1.

Patent document 3 discloses a novel molecule for antisense nucleic acid, artificial nucleoside, with which expression of a specific gene can be efficiently regulated. This nucleoside has a bridged structure of 2',4'-bridged nucleic acid (BNA/LNA) introduced with an amide bond. Since this molecule has binding affinity to a single-stranded RNA comparable to that of the conventional 2'4'-BNA/LNA, and nuclease resistance higher than that of LNA, and it shows very strong binding affinity especially to a single-stranded RNA, application thereof to nucleic acid drugs is expected.

Patent document 4 discloses that a siRNA targeted to a predetermined partial sequence of a gene encoding YB-1 induces apoptosis of vascular endothelial cells, and inhibits lumen formation by endothelial cells. Patent document 4 also describes that this siRNA suppresses abnormal vascular neogenesis at tumor sites etc., and destroys hyperplastic neovascularity, and therefore it is effective for intractable tumors and proliferative vascular diseases.

### Prior Art References

### Patent documents

Patent document 1: Japanese Patent Unexamined Publication (KOKAI) No. 2011-88876
Patent document 2: U.S. Patent No. 6,140,126
Patent document 3: International Patent Publication WO2011/052436
Patent document 4: Japanese Patent Unexamined Publication (KOKAI) No. 2013-216627

### Summary of the Invention

### Object to be Achieved by the Invention

It is known that use of siRNA or miRNA as a method for inhibiting expression of a target gene can provides higher suppression effect compared with use of an antisense nucleic acid. However, siRNA and miRNA have drawbacks that they are quickly decomposed by a decomposition enzyme, nuclease, i.e., suffer from low stability, in the body, and since siRNA and miRNA are double-stranded nucleic acids, a molecule thereof alone is not taken up into cells, and thus they require a delivery device. However, any effective delivery device for nucleic acids has not been developed.

On the other hand, although an antisense nucleic acid constituted by the artificial nucleic acid, LNA, in which sugar moiety is appropriately bridged, shows nuclease resistance, and is stable in living bodies, risk of toxicity thereof to the liver and kidney has been reported. An artificial nucleic acid of a new generation showing increased nuclease resistance and complementary nucleic acid-binding ability compared with the aforementioned LNA has also been recently developed, and effects thereof for suppressing expression of hyperlipidemia factor in the liver, and improving the symptoms have been reported.

However, any artificial antisense nucleic acid structure of which antitumor effect and safety in living bodies are secured does not exist yet.

If the malignant alteration factor, Y-box binding protein 1 (YB-1), which is overexpressed in cancer cells and tumor neovascularity, can be suppressed in living bodies to inhibit increase and metastasis of tumors, highly flexible cancer therapies are enabled, but there has not been found any target gene expression-suppressing nucleic acid structure such as siRNA and antisense nucleic acid that can function in living bodies even without any delivery device. Any useful delivery device for nucleic acids has also not been reported yet.

An object of the present invention is to provide an artificial nucleic acid antisense anti-malignant tumor agent that can exhibit an effect against a wide range of malignant tumors in living bodies without any delivery device.

### Means for Achieving the Object

The present invention provides the followings.
[1] An oligonucleotide having a 13- to 17-nucleotide length, which comprises a part of the sequence of SEQ ID NO: 1 or 2 having at least 8-nucleotide length, and is targeted to a polynucleotide encoding human YB-1, namely, regulates expression of YB-1.
[2] The oligonucleotide according to [1], which has a 14- to 16-nucleotide length.
[3] The oligonucleotide according to [2], which has 15-nucleotide length.
[4] An oligonucleotide consisting of the sequence of SEQ ID NO: 1 or 2.
[5] The oligonucleotide according to any one of [1] to [4], which comprises at least one bridged nucleic acid.
[6] The oligonucleotide according to [5], wherein the bridged nucleic acid has a nucleoside structure selected from the group consisting of the following nucleoside structures: 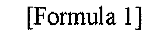 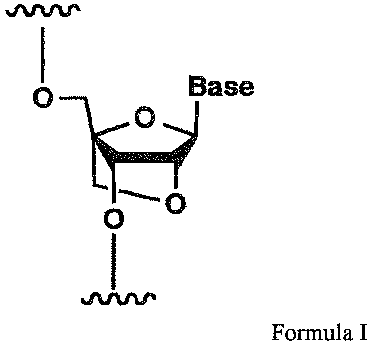 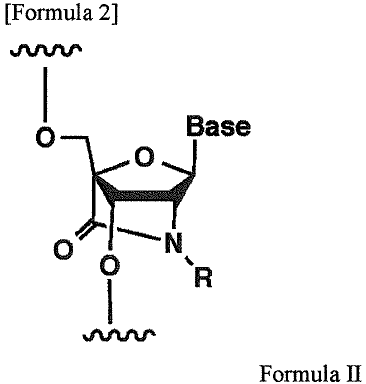 (in the formulas I and II:
   Base represents a purin-9-yl group or 2-oxo-1,2-dihydropyrimidin-1-yl group which may have one or more arbitrary substituents selected from group α, wherein the group α consists of hydroxyl group, a hydroxyl group protected with a protective group for nucleic acid synthesis, a linear alkyl group having 1 to 6 carbon atoms, a linear alkoxy group having 1 to 6 carbon atoms, mercapto group, a mercapto group protected with a protective group for nucleic acid synthesis, a linear alkylthio group having 1 to 6 carbon atoms, amino group, a linear alkylamino groups having 1 to 6 carbon atoms, an amino group protected with one or more protective groups for nucleic acid synthesis, and a halogen atom; and
   R represents hydrogen atom, an alkyl group having 1 to 7 carbon atoms, which may be branched or form a cyclic group, an alkenyl group having 2 to 7 carbon atoms, which may be branched or form a cyclic group, an aryl group having 3 to 12 carbon atoms, which may have one or more substituents selected from the group α, and may contain a heteroatom, an aralkyl group including an aryl moiety having 3 to 12 carbon atoms, which may have one or more substituents selected from the group α, and may contain a heteroatom, or a protective group for amino group used in nucleic acid synthesis.
[7] The oligonucleotide according to any one of [1] to [6], which contains at least one phosphorothioate type nucleotide.
[8] A method for suppressing expression of YB-1 in a cell or tissue, which comprises the step of contacting the oligonucleotide according to any one of [1] to [7] with the cell or tissue.
[9] A method for treating a disease or condition relevant to expression of YB-1, which comprises the step of administrating the oligonucleotide according to any one of [1] to [7] to an object.
[10] The method according to [9], wherein the disease or condition relevant to expression of YB-1 is a cancer.
[11] The method according to [10], wherein the cancer is gastric cancer, large intestine cancer, esophageal cancer, breast cancer, pancreatic cancer, biliary tract cancer, lung cancer, malignant mesothelioma, or ovarian cancer.
[12] The method according to [10] or [11], wherein the cancer is an anticancer agent-resistant cancer.
[13] The method according to [12], wherein the anticancer agent-resistant cancer is gemcitabine-resistant pancreatic cancer.
[14] A pharmaceutical composition containing the oligonucleotide according to any one of [1] to [7], and a pharmaceutically acceptable carrier.
[15] The pharmaceutical composition according to [14], which is for treating a disease or condition relevant to expression of YB-1.
[16] The pharmaceutical composition according to [15], which is for treating a cancer.
[17] The pharmaceutical composition according to [16], which is for treating gastric cancer, large intestine cancer, esophageal cancer, breast cancer, pancreatic cancer, biliary tract cancer, lung cancer, malignant mesothelioma, or ovarian cancer.
[18] The pharmaceutical composition according to [16] or [17], which is for treating an anticancer agent-resistant cancer.
[19] The pharmaceutical composition according to [18], which is for treating gemcitabine-resistant pancreatic cancer.
[20] The pharmaceutical composition according to any one of [14] to [19], which is for subcutaneous administration or oral administration.

### Effect of the Invention

According to the present invention, there is provided an oligonucleotide with which expression of YB-1 can be efficiently regulated.

### Brief Description of the Drawings

[Fig. 1] YB-1 is overexpressed in cancer cells and neovascularity of pancreatic cancer and biliary tract cancer.
[Fig. 2] Proliferation-suppressing effect of YB-1 antisense oligo BNA.
[Fig. 3] Suppression of cell cycle and induction of apoptosis by YB-1 antisense oligo BNA.
[Fig. 4] Antitumor effect attained by local administration of YB-1 antisense oligo BNA.
[Fig. 5] Antitumor effect attained by administration of YB-1 antisense oligo BNA into blood.
[Fig. 6] Administration of YB-1 antisense oligo BNA into blood suppresses expression of YB-1 in tumor, liver, and kidney.
[Fig. 7] Safety evaluation of administration of YB-1 antisense oligo BNA into blood (repetitive administration of artificial nucleic acid (BNA) into blood induces hepatic dysfunction).
[Fig. 8] Evolution of artificial nucleic acid based on modification of bridging structure.
[Fig. 9] Improvement of safety of artificial nucleic acid based on modification of structure, - LNA/BNAvs. BNA^{amide} (AmNA).
[Fig. 10] Antitumor effect attained by administration of artificial nucleic acid, YB-1 antisense oligo AmNA, into blood.
[Fig. 11] Antitumor effect attained by administration of artificial nucleic acid, YB-1 antisense oligo AmNA, into blood (antitumor effect is exhibited against gemcitabine-resistant pancreatic cancer).
[Fig. 12] Comparison of YB-1 expression-suppressing effects of various antisense oligo-nucleic acids. A: MiaPaCa2 (pancreatic cancer cell), B: GBd-15 cell, C: TGBC2 cell.
[Fig. 13A] YB-1 expression-suppressing effect attained by 2 times of administration of ASO_#1_AmN or ASO_#10_AmNA to a HCT116 (large intestine cancer) subcutaneous tumor model. Saline, n=2; Ctrl, n=2; #1 AmNA, n=3; #10 AmNA, n=3.
[Fig. 13B] YB-1 expression-suppressing effect attained by 2 times of administration of ASO_#1_AmN or ASO_#10_AmNA to a SUIT2-GR (pancreatic cancer) subcutaneous tumor model. Saline, n=2; Ctrl, n=2; #1 AmNA, n=3; #10 AmNA, n=3.
[Fig. 13C] YB-1 expression-suppressing effect attained by 4 times of administration of ASO_#10_AmNA to a SUIT2-GR (pancreatic cancer) subcutaneous tumor model.
[Fig. 14] Antitumor effect against pancreatic cancer and large intestine cancer attained by repetitive administration into blood. (A) Suppression of proliferation of subcutaneous tumor, (B) induction of apoptosis of cancer cells, (C) suppression of microvessel density in tumor tissue.
[Fig. 15] Antitumor effect against lung cancer. (A) Photograph of subcutaneous tumor on day 21 of treatment, (B) Growth inhibition of LLC s.c. tumor (* P<0.05, ** P<0.01, n=5).
[Fig. 16] Antitumor effect against malignant mesothelioma. (A) YB-1-KD efficiency, *P<0.001, (B) photographs of malignant mesothelioma cell strain H226 cells, (C) photographs of subcutaneous tumors after treatment are shown in the left panel, and tumor weights on day 21 of the treatment are shown in the right panel (n=3, P<0.05).
[Fig. 17] Antitumor effect against ovarian cancer inoculated on peritoneum. (A) Suppression of YB-1 expression by #10 AmNA YB-1 antisense, (B) suppression of cell proliferation, *P<0.001, (C) comparison of luciferase signals provided by inoculation on peritoneum on day 21 of treatment is shown in the left panel, and tumor growth inhibition (n=4, P<0.05) is shown in the right panel.
[Fig. 18] Antitumor effect against gastric cancer inoculated on peritoneum. (A) Suppression of YB-1 expression by #10 AmNA YB-1 antisense, (B) concentration-dependent suppression of cell proliferation, *P<0.001, (C) comparison of luciferase signals provided by inoculation on peritoneum on day 21 of treatment is shown in the left panel, and Kaplan-Meier survival curve (n=5, P<0.05) is shown in the right panel.
[Fig. 19] Anticancer agent sensitivity-enhancing action of artificial nucleic acid, YB-1 inhibitory antisense. (A) Pancreatic cancer cell strain MIAPaCa-2, (B) ovarian cancer cell strain SKOV-3.
[Fig. 20] Radiation sensitivity-enhancing action of artificial nucleic acid, YB-1 inhibitory antisense.
[Fig. 21] Comparison of YB-1 knockdown efficiencies of antisense nucleic acids created from naturally occurring nucleic acid, LNA/BNA, and AmNA nucleic acid. Vascular endothelial cell strains (HUVEC, HPAEC), and pancreatic cancer cell strain (MiaPaCa2-). *P<0.01 vs. BNA, P<0.0001 vs. NA
[Fig. 22] Comparison of YB-1 knockdown efficiencies of siRNA and AmNA-AON. (A) mRNA level, (B) protein level.
   * P<0.05
[Fig. 23] Comparison of YB-1 knockdown efficiencies of 15-mer to 18-mer.

### Modes for Carrying out the Invention

When a numerical value range is expressed as "X to Y", the range includes the values of X and Y that define the range. The term "treatment" means prophylactic treatment and therapeutic treatment, preferably therapeutic treatment. The object of the treatment may be human or non-human animal (for example, mouse). An expression "A and/or B" means at least one of A and B, namely, only A, only B, or A andB.

### [Target]

The oligonucleotide of the present invention is targeted to a specific nucleic acid molecule, and regulates expression thereof. The target nucleic acid may generally be, for example, a cytogene (or mRNA transcribed from a gene) of which expression is relevant to a specific disease or pathological condition, or a nucleic acid molecule derived from an infectious factor. In the present invention, the target nucleic acid encodes the malignant alteration factor, Y-box binding protein 1 (YB-1), which is overexpressed in cancer cells and tumor neovascularity. The target nucleic acid may be a single-stranded DNA, RNA, or double-stranded DNA. The oligonucleotide of the present invention hybridizes with a target region, segment, or site in the target nucleic acid to regulate expression of YB-1. The regulation may be up-regulation or down-regulation (inhibition) of the expression, preferably down-regulation of the expression. The regulation of the expression can be confirmed at the mRNA level or protein level.

### [Oligonucleotide]

The present invention provides an oligonucleotide that regulates expression of YB-1. The term oligonucleotide means an oligomer or polymer of nucleotides consisting of the same or different 2 to 50 nucleosides bound with phosphodiester linkages or other linkages between nucleosides. The oligonucleotide may be a naturally occurring oligonucleotide or a derivative of a naturally occurring oligonucleotide (also referred to as non-naturally occurring, artificial, or modified oligonucleotide). Examples of such a derivative include, for example, saccharide derivatives having a modified saccharide moiety; thioate derivatives having thioated phosphodiester moiety; phosphorothioate derivatives having phosphorothioate moiety corresponding to the phosphodiester moiety of which oxygen atom is replaced with sulfur atom; ester compounds having an esterified phosphoric acid moiety at the end; and amide compounds having an amidated amino group on the purine base. The oligonucleotide may be a single-stranded DNA or RNA, or a double-stranded DNA or RNA, unless especially indicated. One of the preferred embodiments of the oligonucleotide of the present invention is a naturally occurring or non-naturally occurring single-stranded antisense oligonucleotide. The oligonucleotide may be in the form of a pharmaceutically acceptable salt thereof, unless especially indicated.

The oligonucleotide of the present invention may have, for example, a 13- to 18-nucleotide length, preferably 13- to 17-nucleotide length. In one of the preferred embodiments, it has a 14- to 16-nucleotide length, more preferably 15-nucleotide length. Suppressing expression of YB-1 means to suppress the expression to 75% or less, preferably 70% or less, more preferably 50% or less, further preferably 30% or less, of that observed for an appropriate control (for example, that obtained with an oligonucleotide serving as control, or without any oligonucleotide). The details of the experimental method for evaluating the suppression rate are described in Example 2, and so forth included in this specification.

The oligonucleotide of the present invention contains a part of the sequence of SEQ ID NO: 1 or 2 (preferably SEQ ID NO: 2) having at least 8-nucleotide length. According to one of the preferred embodiments, it contains a part of the sequence of SEQ ID NO: 1 or 2 (preferably SEQ ID NO: 2) having at least 10-nucleotide length, more preferably 12-nucleotide length, further preferably 14-nucleotide length. According to one of the particularly preferred embodiments, it contains the entire sequence of SEQ ID NO: 1 or 2 (preferably SEQ ID NO: 2). Examples of the particularly preferred embodiments of the present invention include a 15-mer containing the total sequence of SEQ ID NO: 2, and a 17-mer containing the total sequence of SEQ ID NO: 2 and another sequence. The sequence of the oligonucleotide showing high YB-1 expression-suppressing effect, which is used in the experiment described in the section of examples, is shown as SEQ ID NO: 2 in Sequence Listing.

The oligonucleotide of the present invention may be modified. That is, it may be a derivative of a naturally occurring oligonucleotide (also referred to as non-naturally occurring or artificial oligonucleotide). As known in this field, nucleotides contain a nucleoside moiety, and a nucleoside consists of a combination of a base and a saccharide. The base moiety of nucleoside is usually a heterocyclic base sometimes referred to as "nucleobase" or simply as "base". Two of the most general kinds of the heterocyclic base are purine and pyrimidine. Nucleotides further contain a phosphate group covalently bonded to the saccharide moiety of nucleoside. To such a nucleoside containing a penta-furanose saccharide, phosphate group can bind to the 2, 3, or 5'-hydroxyl moiety of the saccharide. In the formation of oligonucleotide, the phosphate group covalently bond adjacent nucleosides to form a linear polymer compound. Within the oligonucleotide structure, the phosphate groups are commonly referred to as those forming the internucleoside backbone of the oligonucleotide. The normal linkage or backbone of RNA and DNA is a 3' to 5' phosphodiester linkage.

The oligonucleotide may be modified in the saccharide moiety and/or the internucleoside linkage (backbone).

Examples of the oligonucleotide having a modified saccharide moiety include those known as bridged saccharide moiety type artificial nucleic acid (BNA, Bridged Nucleic Acid). The oligonucleotide of the present invention may be such an oligonucleotide. Examples of the structure contained in the oligonucleotide having a modified saccharide moiety (nucleoside moiety) usable in the present invention are shown below.

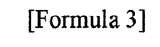

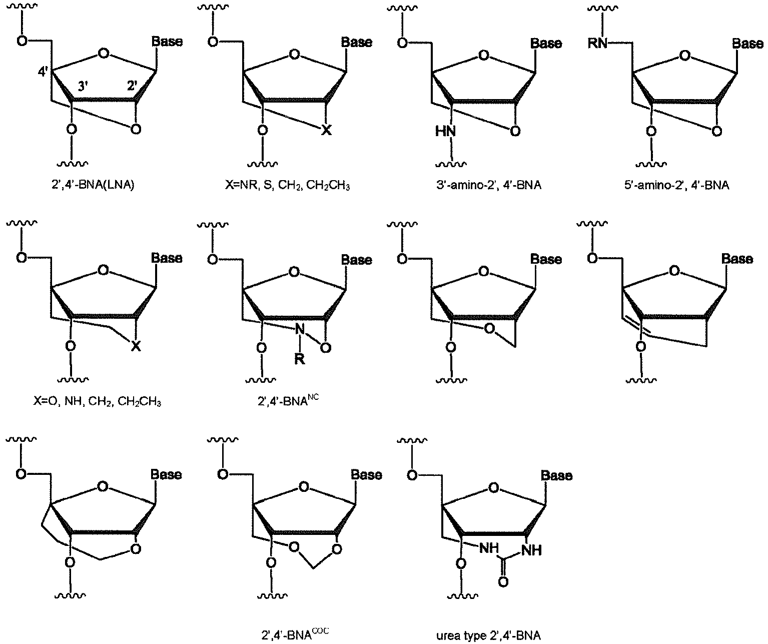

The methods for preparing such oligonucleosides are well known to those skilled in the art. Among those, particularly preferred examples are those comprising the following nucleoside structures.

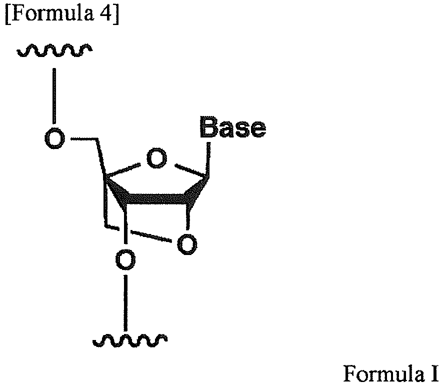

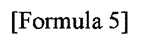

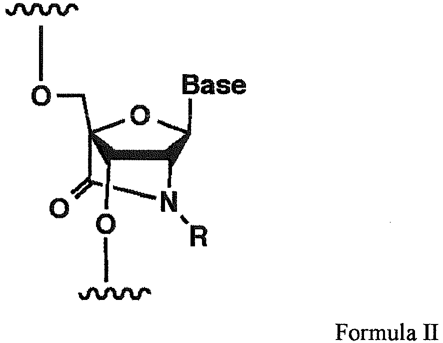

In the aforementioned structures (nucleoside moieties) included in the nucleotides having a modified saccharide moiety usable in the present invention, and the formulas I and II, Base represents a purin-9-yl group or 2-oxo-1,2-dihydropyrimidin-1-yl group which may have one or more arbitrary substituents selected from group α, wherein the group α consists of hydroxyl group, a hydroxyl group protected with a protective group for nucleic acid synthesis, a linear alkyl group having 1 to 6 carbon atoms, a linear alkoxy group having 1 to 6 carbon atoms, mercapto group, a mercapto group protected with a protective group for nucleic acid synthesis, a linear alkylthio group having 1 to 6 carbon atoms, amino group, a linear alkylamino groups having 1 to 6 carbon atoms, an amino group protected with one or more protective groups for nucleic acid synthesis, and a halogen atom.

Base may be 6-aminopurin-9-yl group, 2,6-diaminopurin-9-yl group, 2-amino-6-chloropurin-9-yl group, 2-amino-6-fluoropurin-9-yl group, 2-amino-6-bromopurin-9-yl group, 2-amino-6-hydroxypurin-9-yl group, 6-amino-2-methoxypurin-9-yl group, 6-amino-2-chloropurin-9-yl group, 6-amino-2-fluoropurin-9-yl group, 2,6-dimethoxypurin-9-yl group, 2,6-dichloropurin-9-yl group, 6-mercaptopurin-9-yl group, 2-oxo-4-amino-1,2-dihydropyrimidin-1-yl group, 4-amino-2-oxo-5-fluoro-1,2-dihydropyrimidin-1-yl group, 4-amino-2-oxo-5-chloro-1,2-dihydropyrimidin-1-yl group, 2-oxo-4-methoxy-1,2-dihydropyrimidin-1-yl group, 2-oxo-4-mercapto-1,2-dihydropyrimidin-1-yl group, 2-oxo-4-hydroxy-1,2-dihydropyrimidin-1-yl group, 2-oxo-4-hydroxy-5-methyl-1,2-dihydropyrimidin-1-yl group, or 4-amino-5-methyl-2-oxo-1,2-dihydropyrimidin-1-yl group (also referred to as 5-methylcytosin-1-yl or mC).

R represents hydrogen atom, an alkyl group having 1 to 7 carbon atoms, which may be branched or form a cyclic group, an alkenyl group having 2 to 7 carbon atoms, which may be branched or form a cyclic group, an aryl group having 3 to 12 carbon atoms, which may have one or more substituents selected from the group α, and may contain a heteroatom, an aralkyl group including an aryl moiety having 3 to 12 carbon atoms, which may have one or more substituents selected from the group α, and may contain a heteroatom, or a protective group for amino group used in nucleic acid synthesis.

More preferably, R is hydrogen atom, methyl group, ethyl group, n-propyl group, isopropyl group, phenyl group, or benzyl group, and R is more preferably hydrogen atom or methyl group.

The bridged nucleotide more preferably has the following nucleoside structure.

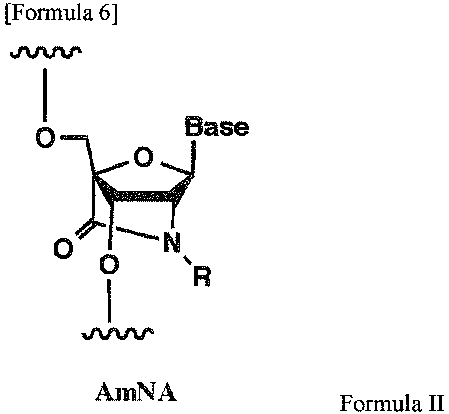

In the formula, Base and R have the same meanings as those defined above.

The bridge of saccharide moiety may be formed in at least one, preferably several (for example, 3 or more, preferably 4 or more, more preferably 5 or 6), of the nucleotides that constitute the oligonucleotide.

The oligonucleotide of the present invention may be an oligonucleotide containing a modified backbone or a non-naturally occurring internucleoside linkage.

Preferred examples of the modified oligonucleotide backbone containing a phosphorus atom include, for example, phosphorothioates, chiral phosphorothioates, phosphorodithioates, phosphotriesters, aminoalkylphosphotriesters, methyl and other alkyl phosphonates including 3'-alkylene phosphonates, 5'-alkylene phosphonates and chiral phosphonates, phosphinates, phosphoramidates including 3'-aminophosphoramidate and aminoalkylphosphoramidates, thionophosphoramidates, thionoalkylphosphonates, thionoalkylphosphotriesters, selenophosphates and boranophosphates having normal 3'-5' linkages, 2'-5' linked analogues of these, and those having inverted polarity wherein one or more internucleotide linkages are 3' to 3', 5' to 5', or 2' to 2' linkages. The oligonucleotide having inverted polarity preferably includes a single 3' to 3' linkage as the 3'-most internucleotide linkage, i.e., a single inverted nucleoside residue which may be abasic (nucleobase is missing, or it has hydroxyl group in place thereof). Those in the form of any one of various salts, mixed salts, and free acids thereof are also included. The methods for preparing such phosphorous-containing linkages as mentioned above are well known to those skilled in the art.

Preferred modified oligonucleotide backbones not containing phosphorus atom have a backbone that is formed by short chain alkyl or cycloalkyl internucleoside linkages, mixed heteroatom and alkyl or cycloalkyl internucleoside linkages, or one or more short chain heteroatomic or heterocyclic internucleoside linkages. Examples of these include those having morpholino linkages (formed in part from the saccharide moiety of nucleoside); siloxane backbones; sulfide, sulfoxide and sulfone backbones; formacetyl and thioformacetyl backbones; methylene formacetyl and thioformacetyl backbones; riboacetyl backbones; alkene-containing backbones; sulfamate backbones; methyleneimino and methylenehydrazino backbones; sulfonate and sulfonamide backbones; amide backbones; and others having mixed N, O, S and CH₂ component moieties. The methods for preparing such oligonucleosides as mentioned above are well known to those skilled in the art.

Particularly preferred embodiments of the oligonucleotides including a modified backbone or non-naturally occurring internucleoside linkage include phosphorothioate type (sulfurized) oligonucleotides. The term phosphorothioate type oligonucleotide refers to an oligonucleotide obtained by substituting sulfur atom for oxygen atom of phosphate group of an oligonucleotide having a phosphodiester linkage. Phosphorothioate type oligonucleotides are preferred, since they show resistance to nucleases. Phosphorothioation (sulfurization) can be performed for a part or all of internucleoside linkages contained in the oligonucleotide.

The oligonucleotide may be modified in both of the saccharide moiety and backbone. Particularly preferred examples of such an oligonucleotide are as follows. Such oligonucleotides can be prepared by those skilled in the art, or since there are many companies that offer contract synthesis services, they can also be prepared by contract synthesis entrusted to such companies.

[Formula 7] T(L) ^{^5}(L) ^{^}T(L) ^{^}c^{^}c^{^}t^{^}g^{^}c^{^}a^{^}c^{^}c^{^5} (L) ^{^}T(L) ^{^}G(L) ^{^}g (N(L)=LNA, ⁵=5-mC, ⁵(L)=LNA_mC, n=DNA, ^{^}=sulfurized

[Formula 8] T (A) ^{^5} (A) ^{^}T (A) ^{^}c^{^}c^{^}t^{^}g^{^}c^{^}a^{^}c^{^}c^{^5} (A) ^{^}T (A) ^{^}G (A) ^{^}g (N(A)=AmNA, ⁵(A)=AmNA_mC, n=DNA,

^{^}=sulfurized, AmNA is the nucleoside structure of the aforementioned formula II wherein R is methyl)

[Formula 9] T (A) ^{^}T (A) ^{^5} (A) ^{^}t^{^}c^{^}c^{^}t^{^}g^{^}c^{^}a^{^}c^{^}c^{^5}(A) ^{^}T (A) ^{^}G(A) ^{^}g G (A) ^{^}T (A) ^{^}T (A) ^{^}c^{^}t^{^}c^{^}c^{^}t^{^}g^{^}c^{^}a^{^}c^{^}c^{^5}(A) ^{^}T (A) ^{^}G (A) ^{^}g T(A) ^{^}G(A) ^{^}T(A) ^{^}t^{^}c^{^}t^{^}c^{^}c^{^}t^{^}g^{^}c^{^}a^{^}c^{^}c^{^5}(A) ^{^}T(A) ^{^}G(A) ^{^}g T(A) ^{^5}(A) ^{^}T(A) ^{^}c^{^}c^{^}t^{^}g^{^}c^{^}a^{^}c^{^}c^{^}c^{^}T(A) ^{^}G(A) ^{^}G(A) ^{^}t T(A) ^{^}T(A) ^{^5}(A) ^{^}t^{^}c^{^}c^{^}t^{^}g^{^}c^{^}a^{^}c^{^}c^{^}c^{^}T(A) ^{^}G(A) ^{^}G(A) ^{^}t G(A) ^{^}T(A) ^{^}T(A) ^{^}c^{^}t^{^}c^{^}c^{^}t^{^}g^{^}c^{^}a^{^}c^{^}c^{^}c^{^}T(A) ^{^}G(A) ^{^}G(A) ^{^}t (N(A)=AmNA, ⁵(A)=AmNA_mC, n=DNA,

^{^}=sulfurized, AmNA is the nucleoside structure of the aforementioned formula II wherein R is methyl)

When the expression "sequence of SEQ ID NO: X" is used, the nucleotide sequence (also referred to as base sequence) thereof may be a sequence exclusively consisting of naturally occurring nucleotides, or may be a sequence containing a derivative of naturally occurring nucleotide (also referred to as modified, non-naturally occurring, or artificial nucleotide). Namely, in the sequences disclosed in this specification, claims, drawings, and sequence listing, when the symbol C or c is used, the base moiety of the nucleotide may be cytosine or a derivative thereof (for example, modified cytosine, specifically, 5-methylcytosine, 2'-O-methylcytosine etc.); the moiety other than the base is not particularly limited, and may be a naturally occurring one, the saccharide moiety may be a bridged type one; and the backbone or internucleoside linkage may be, for example, phosphorothioate type one (also referred to as sulfurized one) corresponding to one having a phosphodiester linkage of which oxygen atom is replaced with sulfur atom. The same shall apply to the symbols "A" or "a", "G" or "g", "T" or "t", and "U", or "u". For nucleotides and nucleotide sequences, "5" refers to 5-methylcytosine, unless especially indicated. For nucleotides and nucleotide sequences, "N" or "n" refers to a nucleotide of which base moiety is adenine, guanine, cytosine, thymidine/uracil, or an unknown or other base (DNA or RNA, preferably DNA), or a base itself, unless especially indicated. When a nucleotide or nucleotide sequence is indicated with a capital letter, it means a base or nucleotide having a bridged type saccharide moiety.

### [Pharmaceutical preparation]

The oligonucleotide of the present invention may be an arbitrary pharmaceutically acceptable salt, ester, or salt of such an ester, or a compound of another arbitrary type that can (directly or indirectly) provide a biologically active metabolic product or residue when it is administered to an animal including human.

The term "pharmaceutically acceptable salt" refers to a physiologically and pharmaceutically acceptable salt of the compound of the present invention, that is, a salt that has the desired biological activity of the original compound, and is not imparted with any undesired toxicological effect. Pharmaceutically acceptable salts of oligonucleotides and preferred examples thereof are well known to those skilled in the art.

Preferred specific examples of pharmaceutically acceptable salts of the oligonucleotide include, but not limited to, (a) a salt formed with a cation, for example, those of sodium, potassium, ammonium, magnesium, calcium, polyamine such as spermine and spermidine, etc., (b) an acid addition salt formed with an inorganic acid such as hydrochloric acid, hydrobromic acid, sulfuric acid, phosphoric acid, and nitric acid, (c) a salt formed with an organic acid such as acetic acid, oxalic acid, tartaric acid, succinic acid, maleic acid, fumaric acid, gluconic acid, citric acid, malic acid, arcorbic acid, benzoic acid, tannic acid, palmitic acid, alginic acid, polyglutamic acid, naphthalenesulfonic acid, methanesulfonic acid, p-toluenesulfonic acid, naphthalenedisulfonic acid, and polygalacturonic acid, and (d) a salt formed with an anion of such an element as chlorine, bromine, and iodine.

The pharmaceutical composition as one aspect of the present invention can be used in various dosage forms and through various administration routes. That is, the pharmaceutical composition of the present invention can be administered through an appropriate route depending on which one of local and systemic treatments is desired, or a part to be treated. The administration may be local or systemic administration, and may be oral or parenteral administration. Examples of parenteral administration include administration via mucus such as those of eye, vagina, rectum, etc.; administration by inhalation or aeration (including, for example, spraying as dry powder or aerosol) into thorax, lung, trachea, or nasal cavity; epidermic administration by application or pasting; subcutaneous administration by injection or embedment; and intravenous, intraarterial, intraperitoneal, intramuscular and intracranial administrations (including intraspinal and intraventricular administrations) by injection or drip infusion.

The object to be treated with the pharmaceutical composition includes human and an animal. The animal may be a mammal such as mouse, rat, dog, guinea pig, primate other than human, cat, and pig. Examples of the primate other than human include monkey and chimpanzee. Other examples include laboratory animals such as mouse, rat, dog, primate other than human, cat, and pig. In a preferred embodiment, the object of the treatment may be human.

The pharmaceutical composition of the present invention can be used for treatment of a disease or condition relevant to expression of YB-1. Specific examples of the disease or condition relevant to expression of YB-1 are cancers. The cancers include gastric cancer, large intestine cancer, esophageal cancer, breast cancer, pancreatic cancer, biliary tract cancer, lung cancer, malignant mesothelioma, and ovarian cancer.

To the pharmaceutical composition of the present invention, an enhancer may be added for promotion of incorporation of the oligonucleotide at the cell level. For example, cationic lipids such as LIPOFECTIN™ reagent (Junichi et al., U.S. Patent No. 5,705,188), cationic glycerol derivatives, and polycationic molecules such as polylysine (Lollo et al., International Patent Publication WO97/30731) can be used.

One of examples of the administration scheme considered to be particularly preferred comprises dissolving lyophilized active ingredient in physiological saline or the like just before the administration, and intravenously, intraperitoneally, or topically administering the solution by injection or drip infusion.

The pharmaceutical composition of the present invention can be used together with other pharmaceutical compositions including chemotherapeutic agents, and can be used for an object that has become resistant to a chemotherapy. Examples of such chemotherapeutic agents include nucleotide analogues. More specifically, examples include purine analogues such as thioguanine, fludarabine phosphate (F-Ara-A, Fludara), and cladribine (2-CdA, Leustatin). Examples also include pyrimidine analogues such as cytarabine (Ara-C, Cylocide) and gemcitabine (GEM, Gemzar). Gemcitabine is used for the treatment of pancreatic cancer. Other examples include daunorubicin, daunomycin, dactinomycin, doxorubicin, epirubicin, idarubicin, esorubicin, bleomycin, mafosfamide, ifosfamide, cytosine arabinoside, bischloroethylnitrosourea, busulfan, mitomycin C, actinomycin D, mithramycin, prednisone, hydroxyprogesterone, testosterone, tamoxifen, dacarbazine, procarbazine, hexamethylmelamine, pentamethylmelamine, mitozantrone, amsacrine, chlorambucil, methylcyclohexylnitrosourea, nitrogen mustard, melphalan, cyclophosphamide, 6-mercaptopurine, 6-thioguanine, cytarabine, 5-azacytidine, hydroxyurea, deoxycoformycin, 4-hydroxyperoxycyclophosphoramide, 5-fluorouracil (5-FU), 5-fluorodeoxyuridine (5-FUdR), methotrexate (MTX), colchicine, taxol, vincristine, vinblastine, ethoposide (VP-16), trimetrexate, irinotecan, topotecan, teniposide, cisplatin, diethylstilbestrol (DES), and so forth.

The pharmaceutical composition may contain a pharmaceutically acceptable carrier in addition to the oligonucleotide as the active ingredient. The pharmaceutical composition may be used together with an anticancer agent. By using the pharmaceutical composition together with an anticancer agent of which action mechanism is different from that of the pharmaceutical composition, improvement in the antitumor effect can be expected.

In another embodiment, the composition of the present invention may contain one or more kinds of oligonucleotides that are targeted to a first nucleic acid, and one or more kinds of additional oligonucleotides that are targeted to a second nucleic acid target. Alternatively, the composition of the invention may contain two or more kinds of oligonucleotides that are targeted to different regions of the same nucleic acid target. Some examples of oligonucleotides are known in the art. Two or more combined compounds may be used together or sequentially.

Administration scheme can be designed depending on severity and responsiveness of the disease condition to be treated, with a course of treatment lasting for several days to several months, or until the disease is cured or ameliorated. Optimal administration schedule can be calculated from measurements of drug accumulation in the body of the patient. Those skilled in the art can easily determine optimum dose, administration method and frequency. Optimum dose may vary depending on relative efficacy of individual oligonucleotides, and can generally be estimated on the basis of EC₅₀ found to be effective in vitro or in vivo in animal models. In general, dose of the active ingredient is 0.01 µg to 100 g per kg of body weight, 0.1 µg to 10 g per kg of body weight, 1.0 µg to 1 g per kg of body weight, 10.0 µg to 100 mg per kg of body weight, 100 µg to 10 mg per kg of body weight, or 1 to 5 mg per kg of body weight, and may be given once or more times daily, weekly, monthly or yearly, or even every 2 to 20 years. Those skilled in the art can easily estimate frequency of the administration on the basis of residence times and concentrations of the drug measured in body fluids or tissues. It may be desirable that, following successful treatment, the patient receives maintenance therapy to prevent recurrence of the disease condition, wherein the oligonucleotide is administered at a maintenance dose, ranging from 0.01 µg to 100 g per kg of body weight, at a frequency of from once or more daily to once every 20 years.

The effect of the treatment with the composition of the present invention can be evaluated by collecting a tissue or body fluid from a patient or object undergoing the treatment. It is known in this field that a biological sample can be collected from a specific tissue without giving any harmful effect to a patient or object. Examples of such a tissue and constituent cells thereof include blood (for example, hematopoietic cells such as human hematopoietic progenitor cells, human hematopoietic stem cells, CD34⁺ cells, and CD4⁺ cells), lymphocytes and other blood cells, bone marrow, breast, uterus cervix, colon, esophagus, lymph gland, muscle, peripheral blood, oral mucus, and skin, but are not limited to these. In another embodiment, examples of the body fluid and constituent cells thereof include blood, urine, sperm, synovia, lymph, and cerebrospinal fluid, but are not limited to these. The tissue or body fluid extracted from a patient can be evaluated on the basis of expression level of a target mRNA or protein. Expression level of an mRNA or protein of another gene known or suspected to be relevant to a specific pathological condition, state, or phenotype can also be evaluated. Such an mRNA level can be measured or evaluated by real-time PCR, Northern blotting, in situ hybridization, or DNA array analysis. Such a protein level can be measured by using or on the basis of ELISA, immunoblotting, quantitative protein analysis, protein activity analysis (for example, caspase activity analysis), immunohistochemistry or immunocytochemistry. The effect of the treatment can also be evaluated by measuring a biomarker relevant to a disease or condition in such a tissue or body fluid collected from a patient or object subjected to the treatment as mentioned above by a clinical method according to a procedure known in this field. Examples of such a biomarker include glucose, cholesterol, lipoprotein, triglyceride, free fatty acid, and other markers of glucose and lipid metabolisms; lipoprotein (a) or Lp(a), or apolipoprotein B; liver transaminase, bilirubin, albumin, blood urea nitrogen, creatine, and other markers of renal and hepatic functions; interleukins, tumor necrotizing factor, intracellular adhesion molecules, c responsive protein, and other inflammation markers; testosterone, estrogen and other hormones; tumor markers; vitamins, minerals, and electrolytes, but are not limited to these.

Hereafter, examples of the present invention will be explained. However, the scope of the present invention is not limited by these examples.

### Examples

In the following examples, BNA refers to LNA/2'4'-BNA unless especially indicated.

### Example 1: Confirmation of YB-1 expression in pancreatic cancer and biliary tract cancer

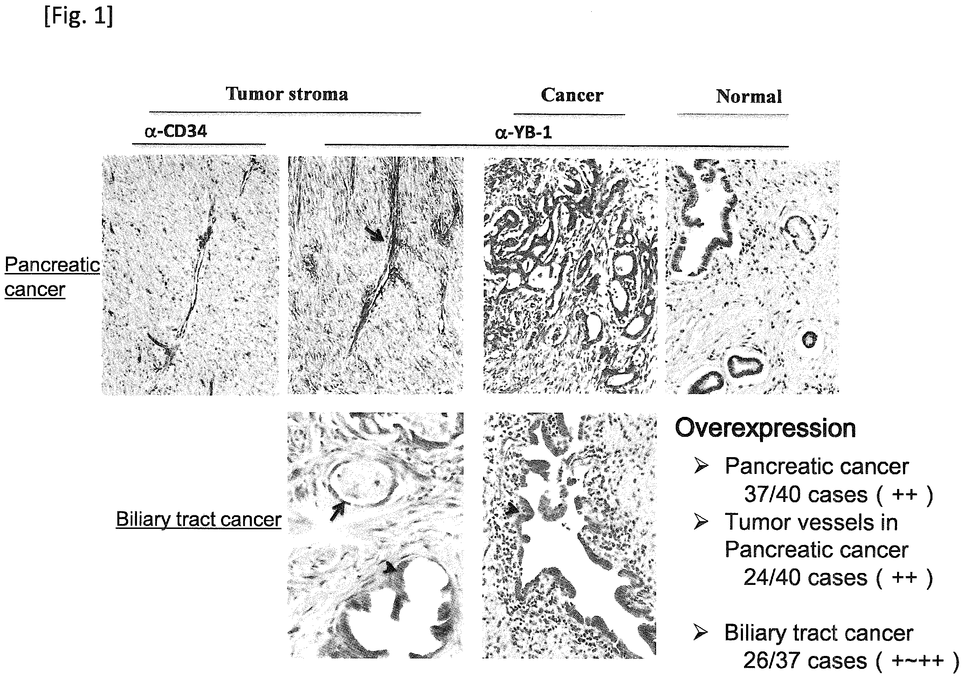

Although it is known that YB-1 involved in anticancer agent and radiation resistances is overexpressed in many cancer species and tumor vessels, expression thereof in pancreatic cancer and biliary tract cancer resistant to an anticancer agent or radiation is not elucidated yet. The inventors of the present invention examined the YB-1 expression in pancreatic cancer and biliary tract cancer by immunostaining using clinical samples (paraffin-embedded sections prepared from formalin-fixed specimens). Overexpression was observed in cells of 37 cases of pancreatic caner out of 40 cases, and overexpression of YB-1 was also observed in tumor vessels (24 cases out of 40 cases), although it was not so frequent as in the cancer cells. Concerning the biliary tract cancer tissues, YB-1 expression in cancer cells was observed at such a medium frequency as 26 cases out of 37 cases. Thus, overexpression of YB-1 in pancreatic and biliary tract cancers was confirmed (Fig. 1).

### Example 2: Screening and evaluation of antisense oligo-nucleic acids

Various candidate antisense oligo-nucleic acids were synthesized, and introduced into various cancer cells by using a gene transduction reagent Lipofectamine RNAiMax, and screening of them was performed on the basis of YB-1 expression-inhibitory ability used as an index to found ASO#1 and ASO#10, which showed knockdown efficiencies of 70% or higher at a concentration of 5 to 20 nM (data are not shown). The structure of ASO#10 is shown below.

[Formula 10] T(L) ^{^5}(L) ^{^}T(L) ^{^}c^{^}c^{^}t^{^}g^{^}c^{^}a^{^}c^{^}c^{^5} (L) ^{^}T(L) ^{^} (L) ^{^}g (N(L)=LNA, ⁵=5-mC, ⁵(L)=LNA_mC, n=DNA, ^{^}=sulfurized

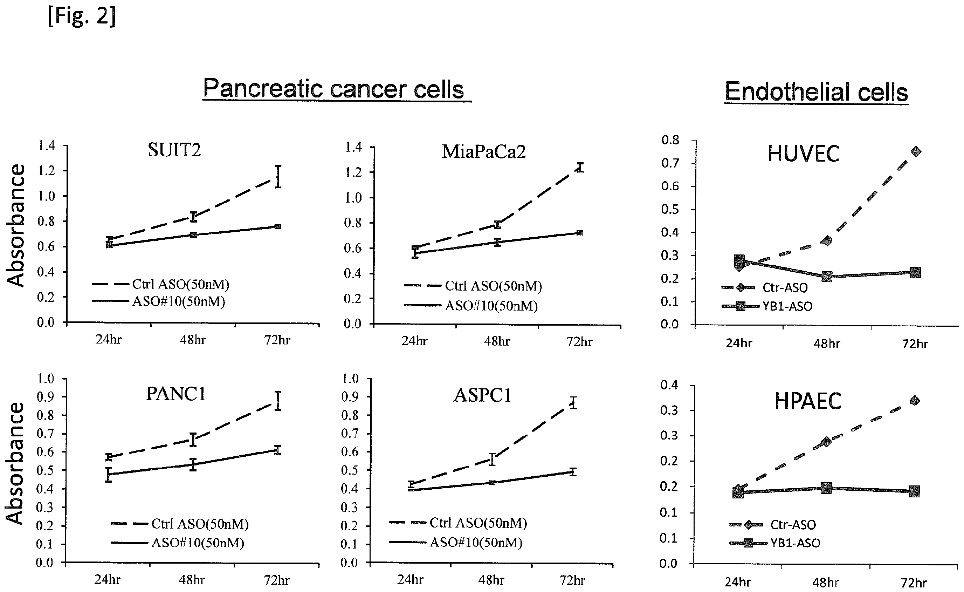

Then, pancreatic cancer and vascular endothelial cell proliferation-suppressing effects of ASO#10 (also referred to as "YB-1 ASO", TCTcctgcaccCTGg, SEQ ID NO: 2) were examined. The cells were inoculated into wells of a 96-well plate at a density of 60% confluent, ASO#10 was introduced into the pancreatic cancer cells at a final concentration of 50 nM, and endothelial cells at a final concentration of 5 nM on the next day by using the gene transduction reagent Lipofectamine RNAiMax, and then the proliferation ability was periodically measured by the MTT assay method. Significantly higher pancreatic cancer cell and endothelial cell proliferation inhibitory effects were observed for ASO#10 synthesized by the inventors of the present invention compared with a control antisense oligo-nucleic acid (CATttcgaagtACTc, SEQ ID NO: 3) (Fig. 2). In addition, it was also confirmed that gastric cancer and breast cancer are also knocked down by ASO#10.

### Example 3: Cell cycle analysis and confirmation of induction of apoptosis using YB-1 ASO BNA

In order to elucidate the mechanism of the pancreatic cancer cell and endothelial cell proliferation inhibitory effect observed in Example 2, YB-1 ASO BNA (TCTcctgcaccCTGg, SEQ ID NO: 2, the parts of the capital letters in the sequence correspond to BNAs, the same shall apply to the following examples) was introduced at a concentration of 5 nM into the endothelial cells (1 x10⁵/well) under the same conditions as mentioned above. After 72 hours from the introduction, the cells were incubated with PI (1 µg/ml) at 37°C for 30 minutes, and then cell cycle analysis was performed by using a flow cytometer (FACS CantoII). Compared with a control antisense oligo-BNA (CATttcgaagtACTc, SEQ ID NO: 3, the parts of the capital letters in the sequence correspond to BNAs, the same shall apply to the following examples), YB-1 ASO provided significant reduction of the G2/M fraction (22.1 vs. 14.5% for HUVEC; 28.5 vs. 14.0% for HPAEC cells), and thus it was found that G2/M arrest was induced. Furthermore, the subG1 fractionation also significantly increased (17.7 vs. 2.0% for HUVEC; 18.7 vs. 1.3% for HPAEC), and thus increase of apoptosis was also observed.

Annexin V labeled with fluorescent FITC was added to the vascular endothelial HUVEC cells, and the fluorescence was observed in order to confirm induction of apoptosis. As a result, increase in Annexin V-positive cells binding to extracellular phosphatidylserine, which is a marker of apoptosis, was observed among the cells treated with YB-1 ASO BNA, whereas Annexin V was hardly observed in the cells treated with the control antisense oligo-BNA.

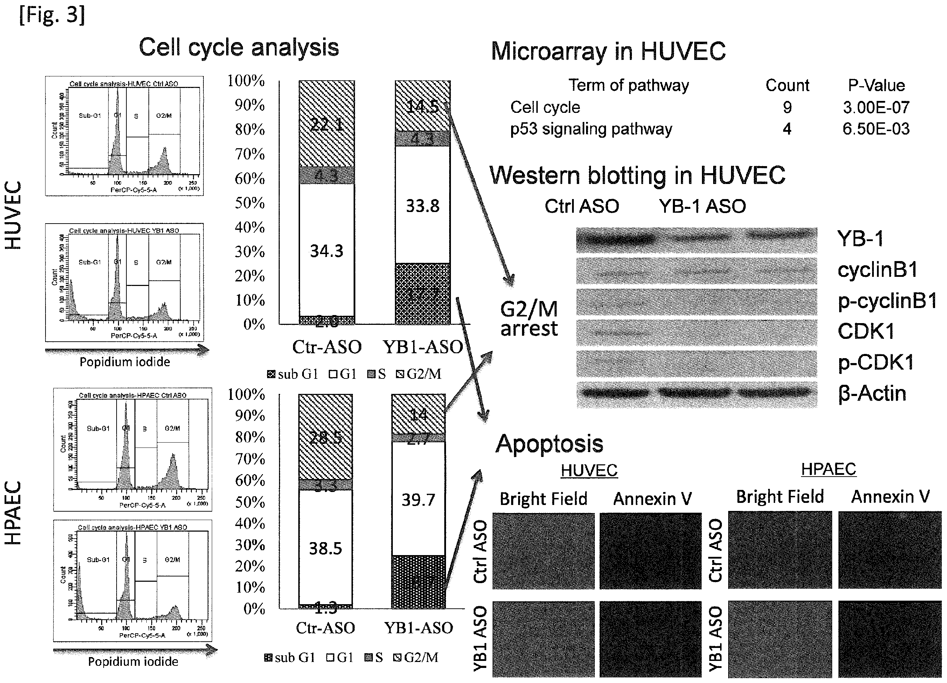

When microarray analysis was performed, suppression of expression of an extensive range of genes relating to cell cycle and cell division was observed for both the HUVEC and HPAEC cells. When proteins were extracted, and expression levels of CDK1 and Cyclin B1 involved in the G2/M transition were compared, it was recognized that the expression was clearly suppressed by YB-1 ASO BNA compared with the control antisense oligonucleotide (Fig. 3).

### Example 4: Effect of local administration of YB-1 ASO BNA

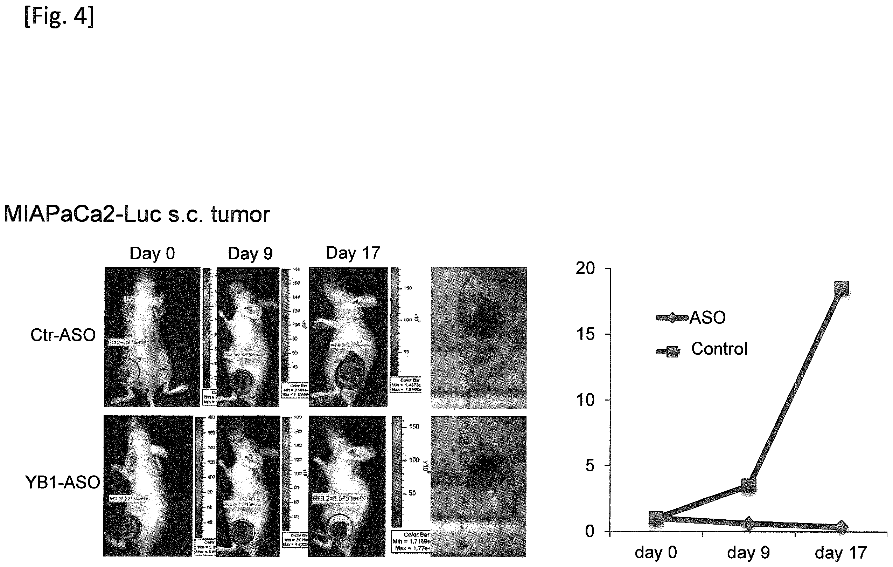

The MiaPaCa-luc pancreatic cancer cells stably expressing luciferase were subcutaneously transplanted into the thighs of nude mice to prepare subcutaneous tumors in a size of 6 to 7 mm. A mixture of YB-1 ASO BNA (final concentration, 10 µM) and atelocollagen implant at a volume ration of 1:1 was locally administered into the tumors (once a week, total twice), and the tumor volume (luciferase activity) was evaluated over time by IVIS imaging. YB-1 ASO BNA provided significant suppression of the tumor volume compared with the control antisense oligo BNA, and regression of the tumors in a white tone was macroscopically observed for the YB-1 ASO BNA group on day 17. On the other hand, increase of the tumor accompanied by flare was observed for the control antisense BNA group (Fig. 4).

### Example 5: Effect of administration of YB-1 ASO BNA into blood

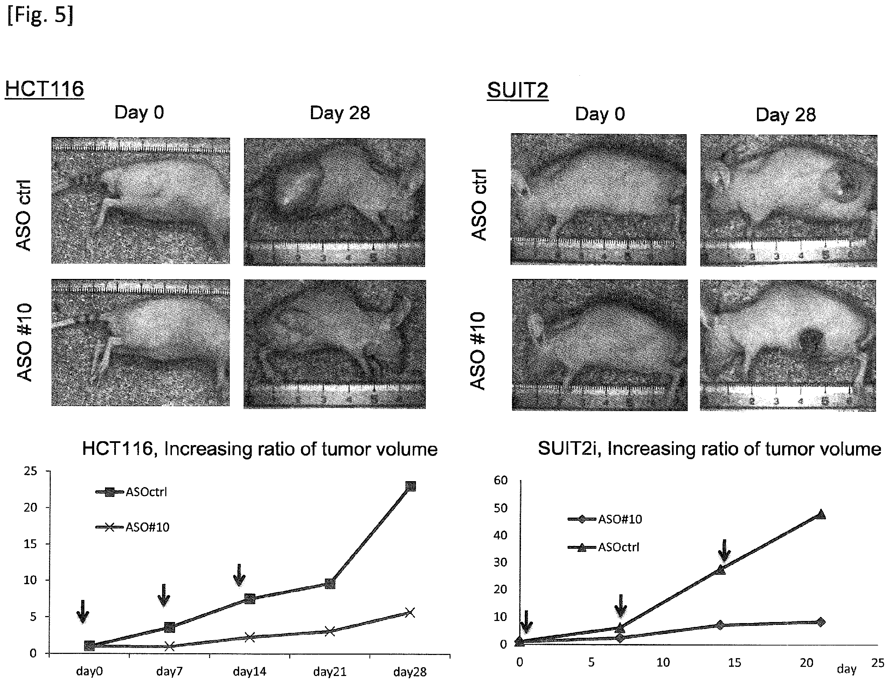

The SUIT2 pancreatic cancer cells or HCT116 large intestine cancer cells were subcutaneously transplanted into the thighs of nude mice to prepare subcutaneous tumors in a size of 6 to 7 mm. YB-1 ASO BNA (200 µg/200 µL) was administered into the blood from the caudal veins (once a week, total 3 times), and the tumor volume was measured over time by measuring the diameter of tumor. YB-1 ASO BNA suppressed proliferation of the tumors to a size of 1/4 to 1/5 of those observed with the control antisense BNA (Fig. 5).

### Example 6: Confirmation of suppression of YB-1 expression by administration of YB-1 ASO BNA into blood

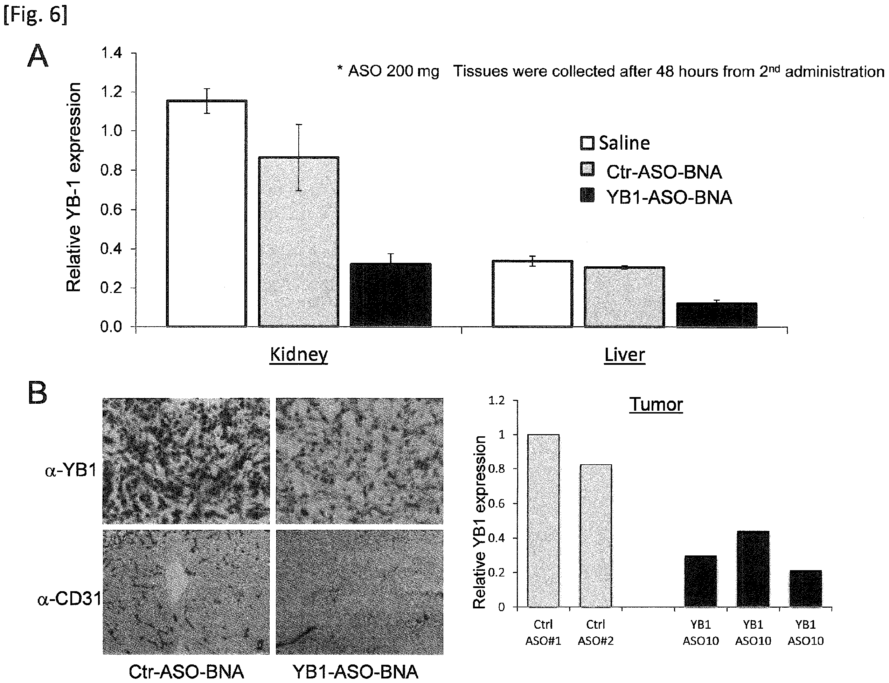

YB-1 ASO BNA (indicated as "ASO#10" in Fig. 6, 200 µg/200 µL) was administered into the blood of the aforementioned nude mice in which subcutaneous tumors were created with the HCT116 large intestine cancer cells from the caudal veins (once a week, total 2 times). After 48 hours from the final administration, subcutaneous tumors, livers, and kidneys were extracted, RNAs were extracted, and YB-1 expression was analyzed by real-time RT-PCR. YB-1 ASO BNA significantly suppressed YB-1 expression in the tumors, livers, and kidneys to about 1/3 of that observed with the control antisense BNA. When YB-1 in frozen sections of the tumors was immunostained, it was found that YB-1 ASO BNA reduced the expression of YB-1 and expression of CD31, which is a neovascularity marker, in the tumor tissues compared with those observed with the control antisense BNA, and thus suppression of YB-1 expression in living bodies by YB-1 ASO BNA could be confirmed (Fig. 6).

### Example 7: Confirmation of safety of administration of YB-1 ASO BNA into blood

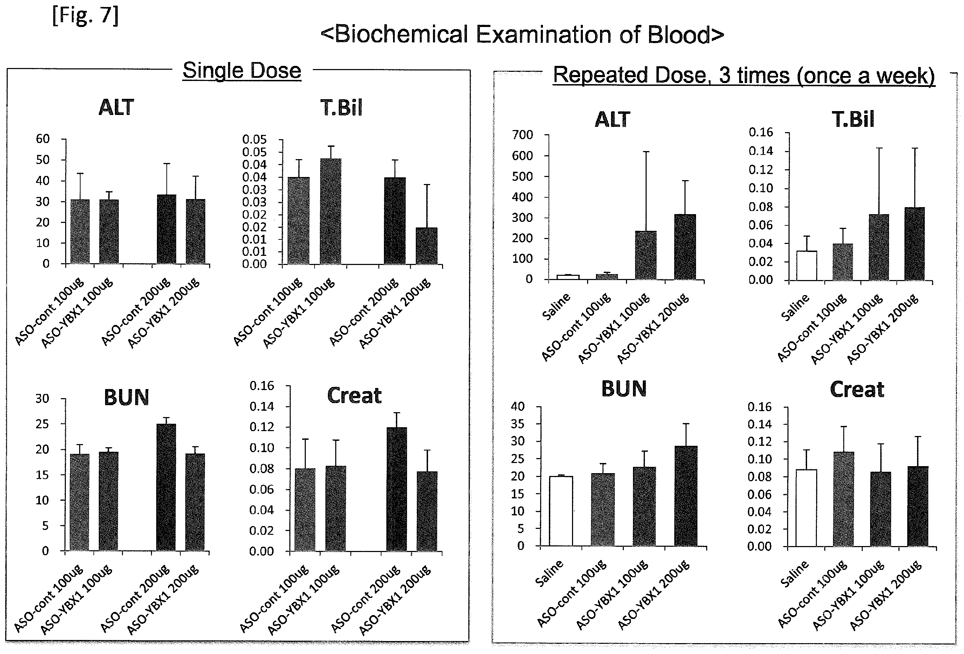

Safety of administration of YB-1 ASO BNA into blood, for which antitumor effect was observed, was examined by blood examination. YB-1 ASO BNA (indicated as "ASO-YBX1" in Fig. 7) at a concentration of 100 µg/200 µL or 200 µg/200 µL was administered once into the blood of nude mice from the caudal veins. After 48 hours, blood was collected, and examined by blood biochemical examinations for ALT, T-Bil, BUN, and creatinin in order to evaluate the liver and kidney functions. As a result, any functional abnormality was not observed for the examination items with both YB-1 ASO BNA and the control antisense BNA. However, when the test substances were repeatedly administered 3 times, only YB-1 ASO BNA showed increases in the liver function markers, AST and T-Bil, whereas the control antisense BNA group and saline control group did not show any functional abnormality (Fig. 7).

### Example 8: Bridged artificial nucleic acid AmNA

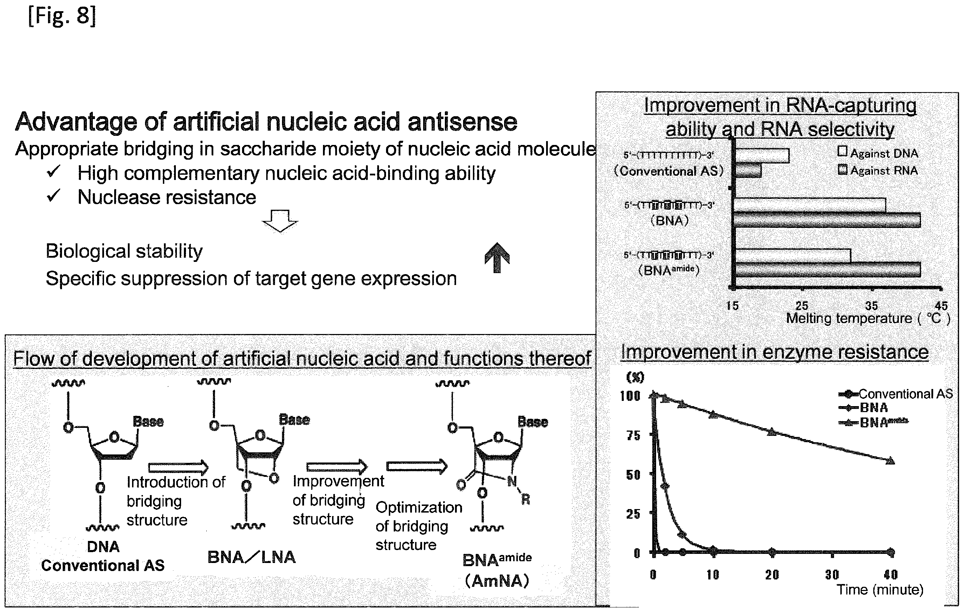

The co-inventors, Obika, Yamamoto, et al. (Osaka University), have advanced development of bridged artificial nucleic acids and optimization of the bridging structure thereof (Fig. 8, lower left). The bridged artificial nucleic acid called AmNA successfully showed reduced binding force to DNA, while maintaining a target RNA-binding ability equivalent to that of 2',4'-BNA/LNA, and thus it is a bridged artificial nucleic acid showing superior RNA selectivity (Fig. 8, upper right). In in-vitro comparative experiments concerning resistance to decomposition by nuclease (3'-exonuclease), it showed higher resistance compared with the relevant bridged artificial nucleic acid developed so far, 2'4'-BNA (Patent document 3 mentioned above).

### Example 9: Influence of administration of YB-1 ASO AmNA into blood on liver function

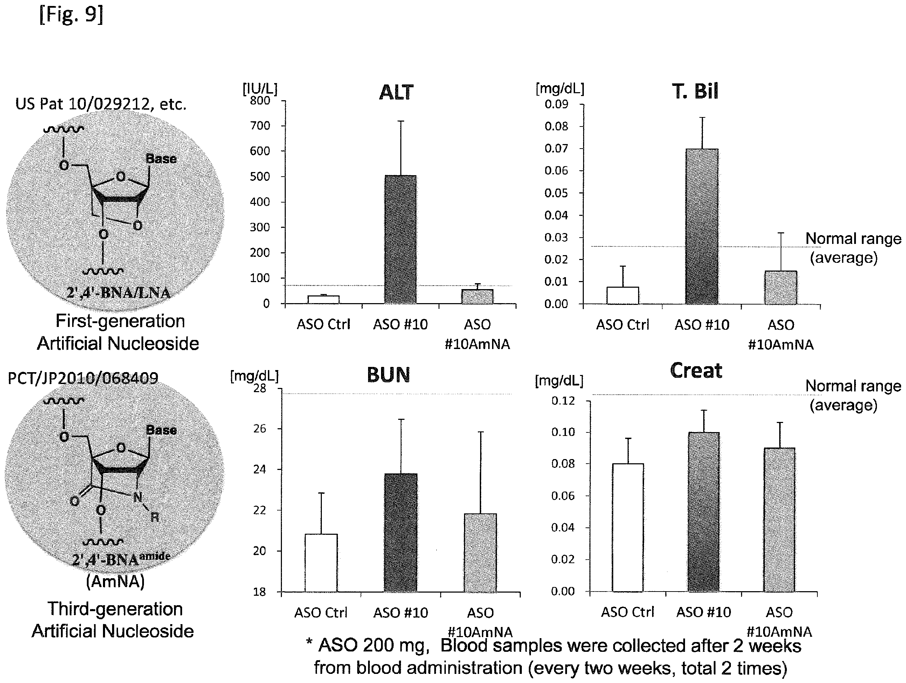

In the same manner as that of Example 7, a solution of 200 µg of oligonucleotide, YB-1 ASO BNA (indicated as "ASO #10" in Fig. 9) or YB-1 ASO AmNA (indicated as "ASO #10 AmNA" in Fig. 9), dissolved in 200 µL of physiological saline was administered into the blood of cancer-bearing nude mice from the caudal veins (once a week, total 3 times). After 48 hours from the final administration, blood was collected, and subjected to the blood biochemical examinations. The structure of YB-1 ASO AmNA is shown below.

[Formula 11] T(A) ^{^5}(A) ^{^}T(A) ^{^}c^{^}c^{^}t^{^}g^{^}c^{^}a^{^}c^{^}c^{^5} (A) ^{^}T(A) ^{^}G(A) ^{^}g (N(A)=AmNA, ⁵(A)=AmNA_mC, n=DNA,

^{^}=sulfurized, AmNA is the nucleoside structure of the aforementioned formula II wherein R is methyl)

YB-1 ASO prepared with 2'4'-BNA/LNA significantly increased AST and ALT, which are hepatic dysfunction markers, compared with the control ASO, whereas YB-1 ASO synthesized with AmNA did not induce hepatic dysfunction (Fig. 9). This result indicates high safety of YB-1 ASO AmNA.

### Example 10: Antitumor effect of administration of YB-1 ASO AmNA into blood

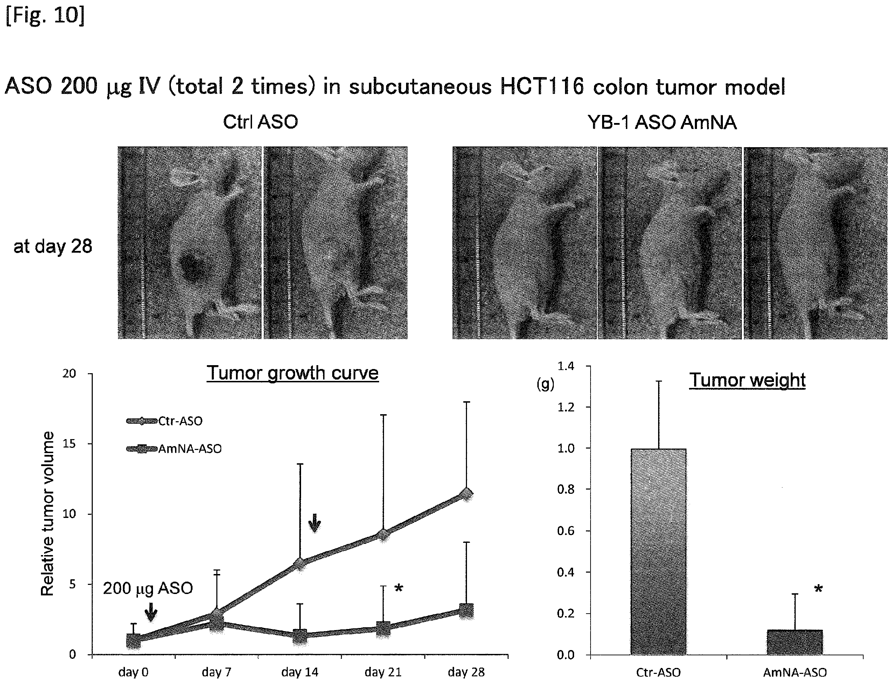

Antitumor effect of administration into blood of YB-1 ASO of AmNA type, of which high safety was confirmed in Example 9 compared with ASO constituted with the conventional BNA, was examined. The human large intestine cancer cells HCT116 were subcutaneously transplanted to the thighs of nude mice. After subcutaneous tumors having a diameter of 6 to 7 mm (average tumor volume, 78 vs. 72 mm³) were formed, 200 µg/200 µL of YB-1 ASO AmNA was administered (once every two weeks, total 2 times) into the blood from the caudal veins. The diameters of the tumors were measured over time, and tumor volumes calculated in accordance with a calculation formula, (Major axis) x (Minor axis)²/2, were compared with those obtained with the control antisense group. The subcutaneous tumors were collected after one week from the second administration (day 28), and weights thereof were compared. In the YB-1 ASO AmNA group, both the tumor diameter and tumor weight were suppressed to about 1/5 of those observed with the control antisense group (Fig. 10, * P<0.05).

### Example 11: Antitumor effect of YB-1 ASO AmNA against anticancer agent-resistant cancer cells

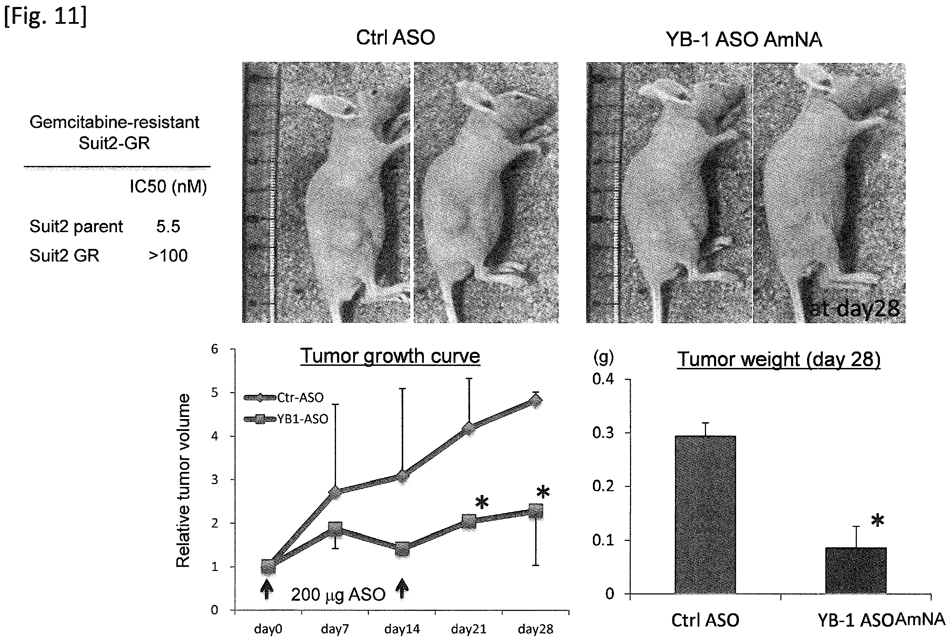

A human pancreatic cancer SUIT2 cell strain was prepared by culturing pancreatic cancer cells resistant to the anticancer agent, gemcitabine, for a long period of time in a medium containing gemcitabine at a low concentration (IC₅₀ value, 100 nM for the resistant strain vs. 5 nM for the parent strain). Subcutaneous tumors were prepared in the same manner as that of Example 10, and 200 µg/200 µL of YB-1 ASO AmNA or control antisense was administered into the blood according to the same protocol (once every two weeks, total 2 times). In the YB-1 ASO AmNA group, both the tumor diameter and tumor weight were suppressed to about 1/3 of those observed for the control antisense group, and thus it was revealed that it shows antitumor effect also on anticancer agent-resistant cancer cells (Fig. 11, *P<0.05)

### Example 12: Comparison of YB-1 expression-suppressing effects of various antisense nucleic acids

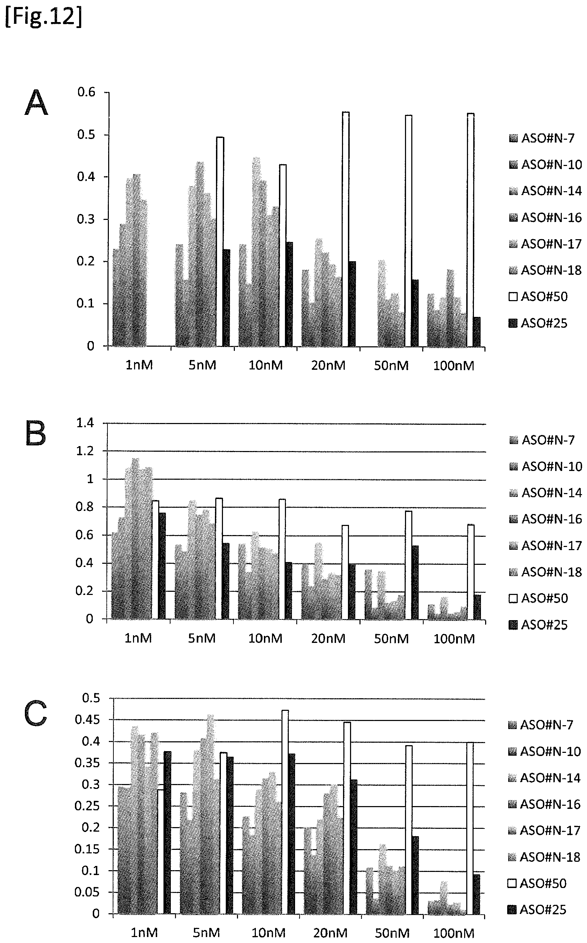

Various antisense nucleic acids were introduced into various cancer cells at predetermined concentrations according to the method used in Example 2, and RNA samples were collected 48 hours afterward, and analyzed by qRT-PCR. The results are shown in Fig. 12.

The sequences of the antisense nucleic acids used in this research were summarized in the following table. HsYBX1-839-BNA18ASO#16, HsYBX1-840-BNA18ASO#17, and HsYBX1-841-BNA18ASO#18 are 18-mers for target sites shifting one nucleotide by one. The nucleotides indicated with the capital letters are BNAs. For example, CCT means ⁵(L)⁵(L)T(L), and ⁵(L) is LNA_mC.

**[Table 1]**

| | | SEQ ID NO.: | NOTE |
|---|---|---|---|
| ASO#N-7 | CCTgcaccctgGTTg | 4 | 15-mer |
| ASO#N-10 | TCTcctgcaccCTGg | 2 | 15-mer |
| ASO#N-14 | TCTcctgcaccctgGTTg | 5 | 18-mer |
| ASO#N-16 | GTTctcctgcacccTGGt | 6 | 18-mer |
| ASO#N-17 | TGTtctcctgcaccCTGg | 7 | 18-mer |
| ASO#N-18 | TTGttctcctgcacCCTg | 8 | 18-mer |
| ASO#N50 | TGCatagataaTGc | 9 | 14-mer |
| ASO#N25 | TCTcctgcaccCTg | 10 | 14-mer |

### Example 13: Confirmation of suppression of YB-1 expression by YB-1 ASO AmNA

In the same manner as that of Example 6, the YB-1 expression-suppressing effect of YB-1 ASO of AmNA type was confirmed. The sequences of the antisense nucleic acids used in this research are summarized in the following table.

**[Table 2]**

| | | SEQ ID NO.: | NOTE |
|---|---|---|---|
| #1AmNA | AGGtgggaaccTTCg | 1 | 15-mer |
| #10AmNA | TCTcctgcaccCTGg | 2 | 15-mer |
| Ctrl | CATttcgaagtACTc | 3 | 15-mer |

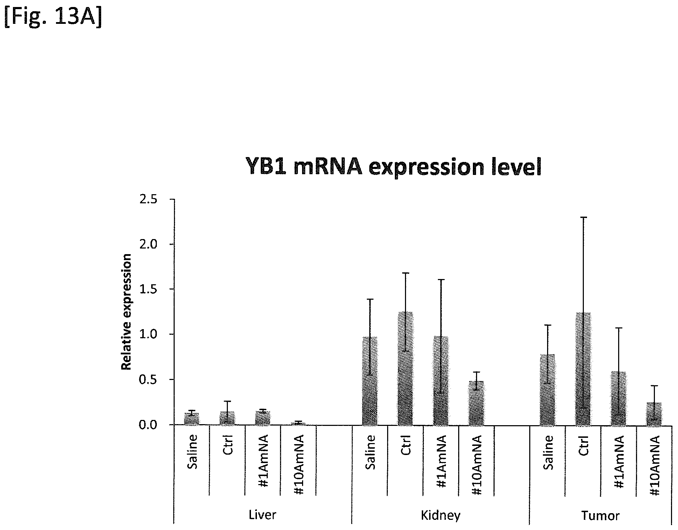

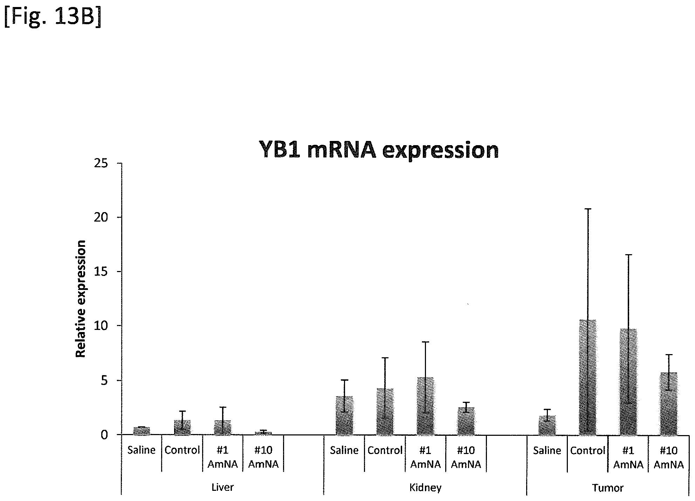

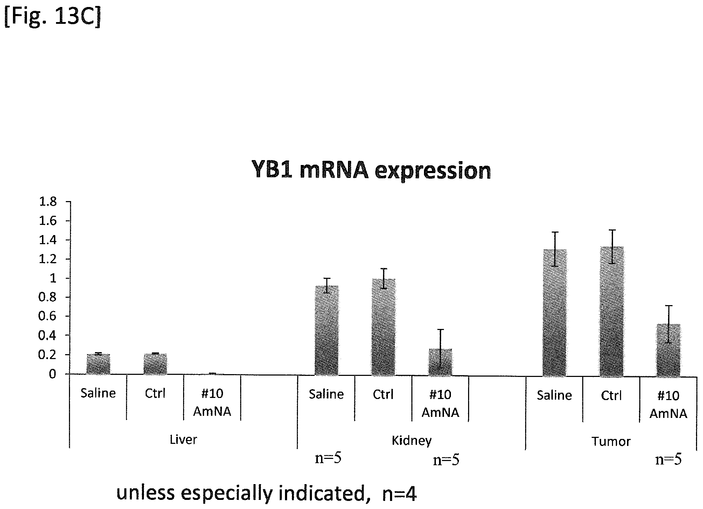

Fig. 13A shows the results of two times of administration (second administration was performed one week after the first administration) of ASO #1 AmNA or #10 AmNA to the HCT116 (large intestine cancer) subcutaneous tumor models. Fig. 13B shows the results of two times of administration (second administration was performed one week after the first administration) of ASO #1 AmNA or #10 AmNA to the SUIT2-GR (pancreatic cancer) subcutaneous tumor models. Fig. 13C shows the results of total 4 times of administration (once a week) of ASO #10 AmNA.

### Example 14: Antitumor effect of repetitive administration into blood against pancreatic cancer and large intestine cancer

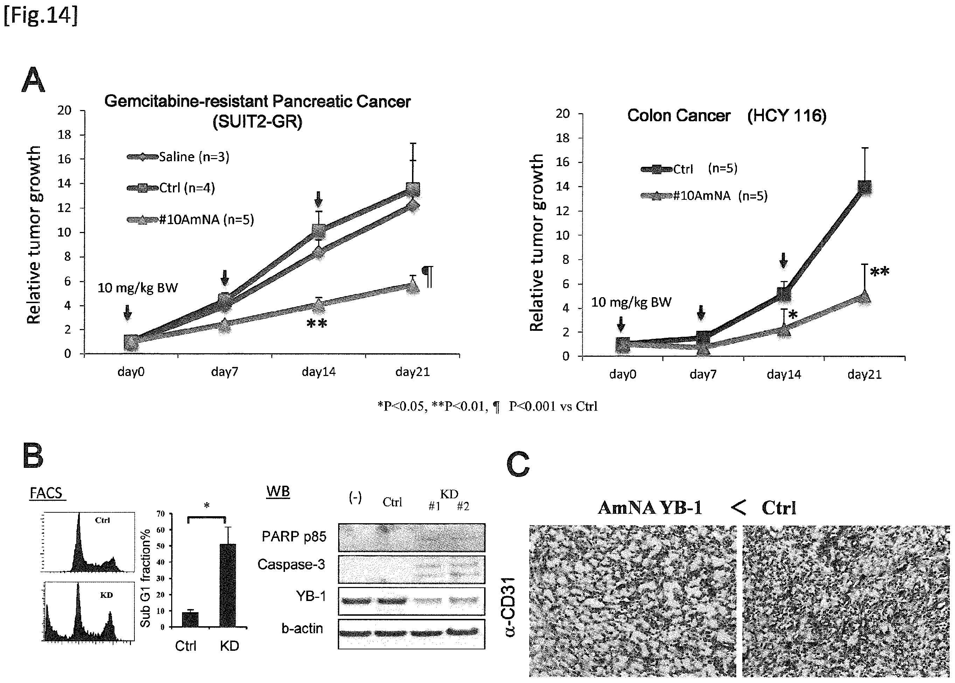

Subcutaneous tumor models were created by subcutaneously transplanting gemcitabine-resistant pancreatic cancer SUIT2-GR cells and large intestine cancer HCT116 cells to the thighs of nude mice. After tumors having a volume of 50 mm³ were formed, 10 mg/kg BW of #10AmNA YB-1 antisense was administered from the caudal veins once a week over three weeks. The tumor diameters were measured over time. Even with the every week administration protocol, any problem was not observed for safety, and significant subcutaneous tumor-suppressing effect was observed on days 14 and 21, as shown in Table 3 (Fig. 14A).

Table 3 shows the results of the measurement performed for blood samples obtained in the treatment experiment. Abnormalities were not observed in the general blood and biochemical examinations.

**[Table 3]**

| **Suit2-GR sc Tumor** | | | | | | |
|---|---|---|---|---|---|---|
| | | WBC (x10²/µl) | RBC (x10⁴/µl) | HGB (g/dL) | HCT (%) | PLT (x10⁴/µl) |
| | Saline | 43 ± 5 | 752 ± 22 | 11.9 ± 0.5 | 37.3 ± 1.4 | 94.2 ± 27.0 |
| | Ctrl | 29 ± 12 | 779 ± 39 | 12.2 ± 0.6 | 38.5 ± 2.1 | 100.5 ± 33.3 |
| | #10AtnNA | 54 ± 14 | 741 ± 48 | 11.8 ± 09 | 37.4 ± 2.4 | 90.4 ± 26.9 |
| | | BUN (mg/dL) | CRE (mg/dL) | AST (IU/L) | ALT (IU/L) | T-BIL (mg/dL) |
| | Saline | 32.4 ± 3.7 | 0.10 ± 0.01 | 147 ± 103 | 37 ± 15 | 0.02 ± 0.01 |
| | Ctrl | 27.9 ± 5.4 | 0.10 ± 0.03 | 170 ± 183 | 45 ± 19 | 0.03 ± 0.01 |
| | #10AmNA | 24.6 ± 3.5 | 0.09 ± 0.01 | 158 ± 71 | 58 ± 29 | 0.03 ± 0.02 |

| **HCT116 sc Tumor** | | | | | | |
|---|---|---|---|---|---|---|
| | | WBC (x10²/ul) | RBC (x10⁴/ul) | HGB (g/dL) | HCT (%) | PLT (x10⁴/ul) |
| | Ctrl | 48 ± 8 | 715 ± 55 | 13.1 ± 03 | 35.7 ± 2.7 | 136.2 ± 10.8 |
| | #10AmNA YB-1 | 39 ± 9 | 696 ± 43 | 13.0 ± 0.6 | 35.5 ± 2.3 | 123.7 ± 40.5 |
| | | | | | | |
| | | BUN (mg/dL) | CRE (mg/dL) | AST (IU/L) | ALT (IU/L) | T-BIL (mg/dL) |
| | Ctrl | 21.2 ± 2.0 | 0.07 ± 0.03 | 80 ± 10 | 26 ± 3 | 0.06 ± 0.02 |
| | #10AmNAYB-1 | 17.4 ± 2.6 | 0.08 ± 0.04 | 148 ± 39 | 77 ± 19 | 0.06 ± 0.02 |

| | | | | | | |
|---|---|---|---|---|---|---|
| AmNA-YB-1 or control AONs (10mg/kg BW) were intravenously administered to nude mice harboring subcutaneous Suit2-GR or HCT116 tumors once a week for three weeks. Blood samples were collected one week after the final treatment of AONs, and subjected to the blood examinations. | | | | | | |

In the same manner as that of Example 3, 20 nM of #10AmNA YB-1 antisense was introduced into the cells of the pancreatic cancer cell strain SUIT2 in vitro. Induction of apoptosis was observed for the pancreatic cancer cells as determined by flow cytometer (FACS) analysis and Western blotting analysis for the apoptosis markers, PARPp85 and caspase-3 (Fig. 14B).

### Suppression of microvessel density in tumor tissue

The neovascularity marker CD31 in the SUIT2-GR subcutaneous tumor of the treatment experiment (A) was immunostained. As a result, it was observed that the microvessel density was decreased by the administration of #10 AmNAYB-1 antisense into the blood, and thus inhibition of neovascularization was induced (Fig. 14C).

### Example 15: Antitumor effect against lung cancer

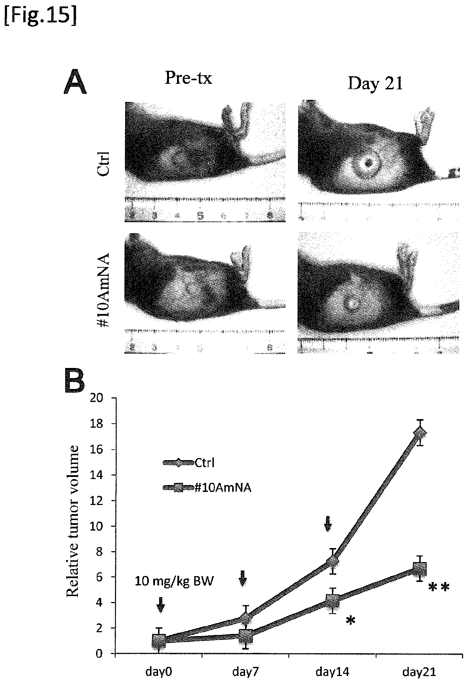

Subcutaneous tumor models of lung cancer, Lewis lung cancer, were created. After tumors having a volume of 50 mm³ were formed, 10 mg/kg BW of #10 AmNA YB-1 antisense was administered from the caudal veins once a week for three weeks. The tumor diameters were measured over time. As a result, subcutaneous tumors of reduced sizes were observed for the #10 AmNA YB-1 antisense administration group compared with the control group on day 21 of the treatment as shown in the photographs (Fig. 15A). On days 14 and 21 of the treatment, increase of the tumor was significantly suppressed in the #10 AmNAYB-1 antisense administration group compared with the control antisense group (Fig. 15B, *P<0.05, ** P<0.01, n=5)

### Example 15: Antitumor effect against malignant mesothelioma

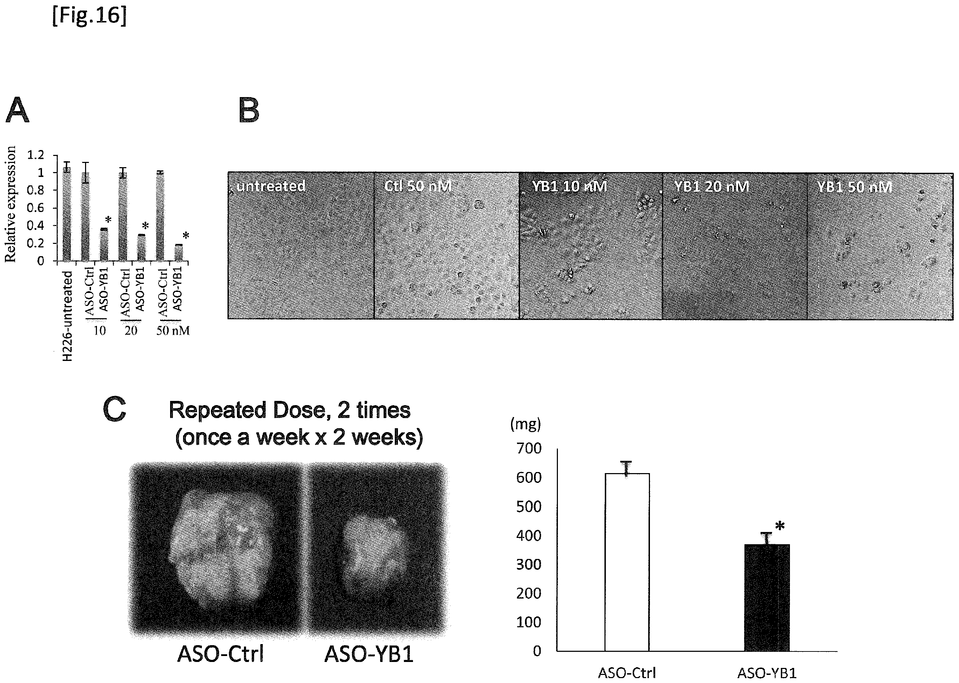

The #10 AmNA YB-1 antisense was introduced into cells of the malignant mesothelioma cell strain H226 using the reagent Lipofectamine RNAiMax, and expression of YB-1 was examined by real-time RT-PCR. Concentration-dependent suppression of YB-1 expression Fig. 16A and suppression of proliferation Fig. 16B were observed with #10 AmNAYB-1 antisense (* P<0.001).

Subcutaneous tumor models of the malignant mesothelioma cell strain H226 were prepared. After tumors having a volume of 50 mm³ were formed, 10 mg/kg BW of #10 AmNA YB-1 antisense was administered from the caudal veins once a week for two weeks, and the tumor diameters were measured over time. The results are shown in Fig. 16C. A photograph of the subcutaneous tumor after the treatment is shown in the left panel. As shown by the right panel, increase of the tumor in terms of tumor weight was significantly suppressed in the #10 AmNA YB-1 antisense blood administration group compared with the control group (n=3, P<0.05).

### Example 16: Antitumor effect on malignant mesothelioma

The #10 AmNAYB-1 antisense was introduced into cells of the malignant mesothelioma cell strain H226 using the reagent Lipofectamine RNAiMax, and expression of YB-1 was examined by real-time RT-PCR. Concentration-dependent suppression of YB-1 expression (A) and suppression of proliferation (B) were observed with #10 AmNA YB-1 antisense (* P<0.001).

Subcutaneous tumor models of the malignant mesothelioma cell strain H226 were prepared. After tumors having a volume of 50 mm³ were formed, 10 mg/kg BW of #10 AmNAYB-1 antisense was administered from the caudal veins once a week for two weeks, and the tumor diameters were measured over time. The results are shown in Fig. 16C. A photograph of the subcutaneous tumor after the treatment is shown in the left panel. As shown in the right panel, increase of the tumor in terms of tumor weight was significantly suppressed in the #10 AmNAYB-1 antisense blood administration group compared with the control group (n=3, P<0.05).

### Example 17: Antitumor effect against ovarian cancer inoculated in peritoneum

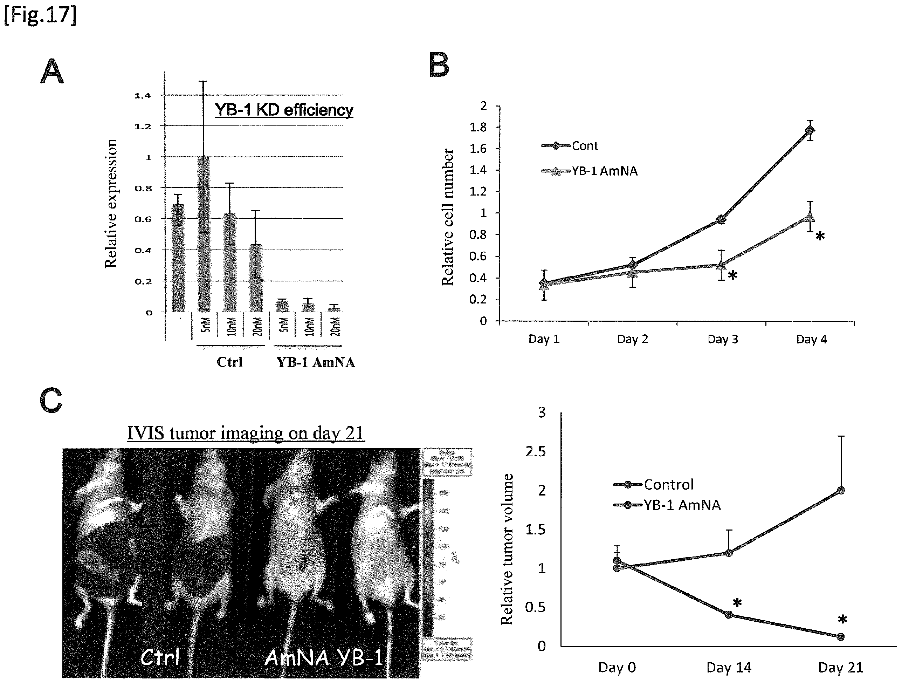

The #10 AmNAYB-1 antisense was introduced into cells of the ovarian cancer cell strain SKOV3 using the reagent Lipofectamine RNAiMax, and expression of YB-1 was examined by real-time RT-PCR. Concentration-dependent suppression of YB-1 expression (Fig. 17A) and suppression of proliferation (Fig. 17B) were observed with #10 AmNA YB-1 antisense (* P<0.001).

Peritoneum inoculation models of ovarian cancer cell strain SKOV3 stably expressing luciferase were prepared. Then, after 1 week from the transplantation of the cancer cells, 10 mg/kg BW of #10 AmNA YB-1 antisense was intraperitoneally administered once a week for two weeks, and the tumor volume was evaluated over time in terms of the luciferase activity. The results are shown in Fig. 17C. The left panel shows comparison of the luciferase signals of the cancer cells inoculated on the peritoneum on day 21 of the treatment. As shown in the right panel, the tumor volume was significantly suppressed in the #10 AmNA YB-1 antisense blood administration group compared with the control group (n=4, P<0.05).

### Example 18: Antitumor effect against gastric cancer inoculated in peritoneum

The #10 AmNA YB-1 antisense was introduced into cells of the gastric cancer cell strain AS44 using the reagent Lipofectamine RNAiMax, and expression of YB-1 was examined by real-time RT-PCR. Concentration-dependent suppression of YB-1 expression (A) and suppression of proliferation (B) were observed with #10 AmNA YB-1 antisense (* P<0.001).

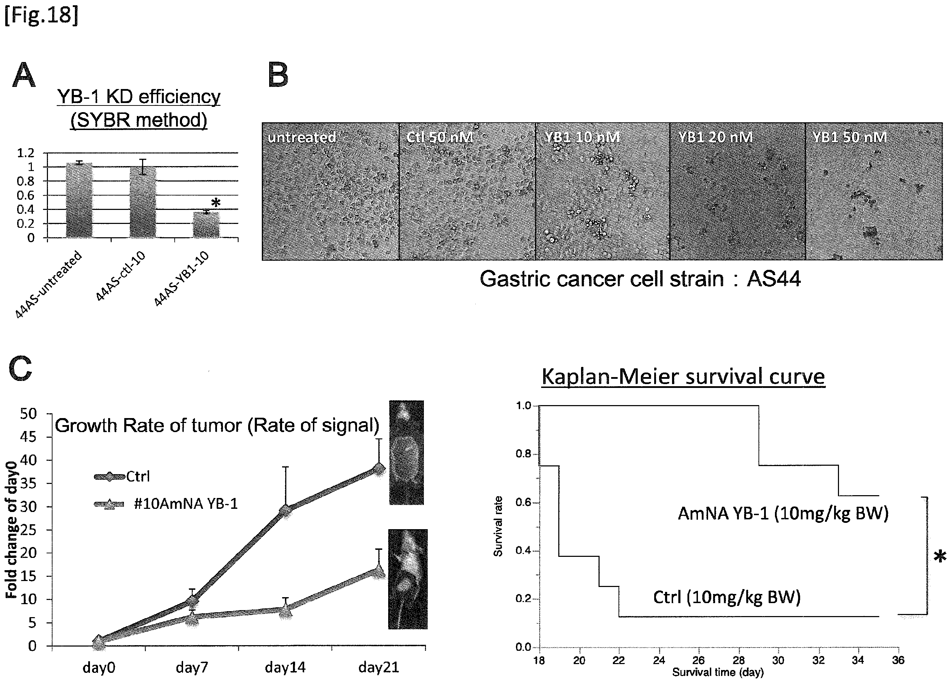

Peritoneum inoculation models of gastric cancer cell strain AS44-luc stably expressing luciferase were prepared. Then, after 1 week from the transplantation of the cancer cells, 10 mg/kg BW of #10 AmNA YB-1 antisense was intraperitoneally administered once a week for two weeks, and the tumor volume was evaluated over time in terms of the luciferase activity. The results are shown in Fig. 18C. The left panel shows comparison of the luciferase signals of the cancer cells inoculated on the peritoneum on day 21 of the treatment. As shown by the Kaplan-Meier survival curve of the right panel, the survival rate was significantly increased in the #10 AmNA YB-1 antisense blood administration group compared with the control group (n=5, P<0.05).

### Example 19: Anticancer agent sensitivity-enhancing effect of artificial nucleic acid, YB-1 inhibitory antisense

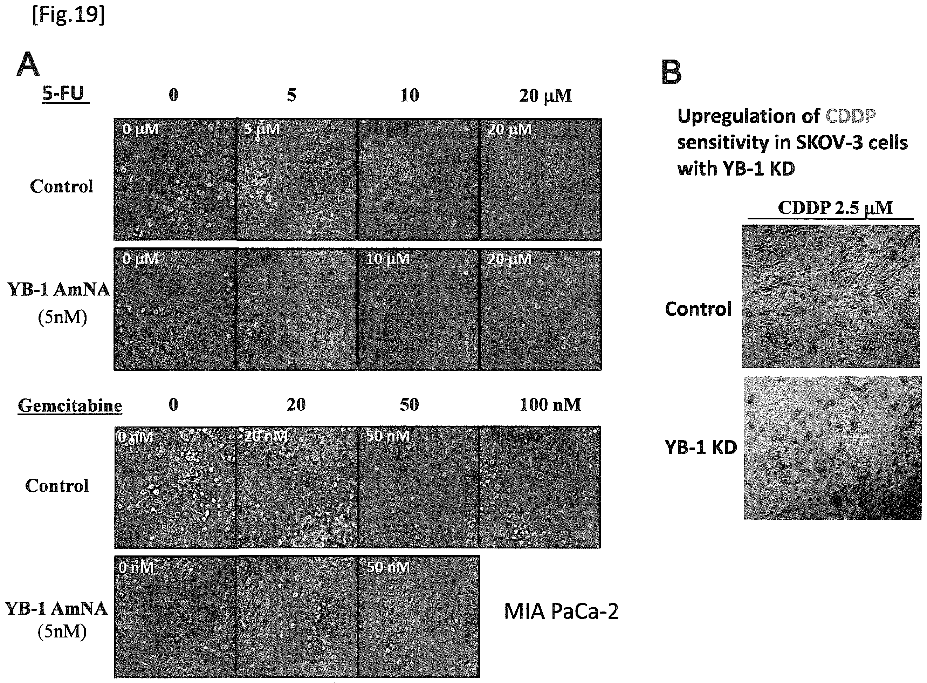

The #10 AmNA YB-1 antisense (5 nM) was introduced into cells of the pancreatic cancer cell strain MIA PaCa-2 (Fig. 19A) and ovarian cancer cell strain SKOV-3 (Fig. 19B) using the reagent Lipofectamine RNAiMax, and cell-killing effects of the anticancer agent observed in the presence and absence of the antisense were compared. Enhancement of the cell-killing effect by the introduction of the #10 AmNAYB-1 antisense at a low concentration was observed. The anticancer agent concentrations that provided equivalent cell-killing effects are shown in blue color (5-FU) or red color (gemcitabine).

### Example 20: Radiation sensitivity-enhancing effect of artificial nucleic acid, YB-1 inhibitory antisense

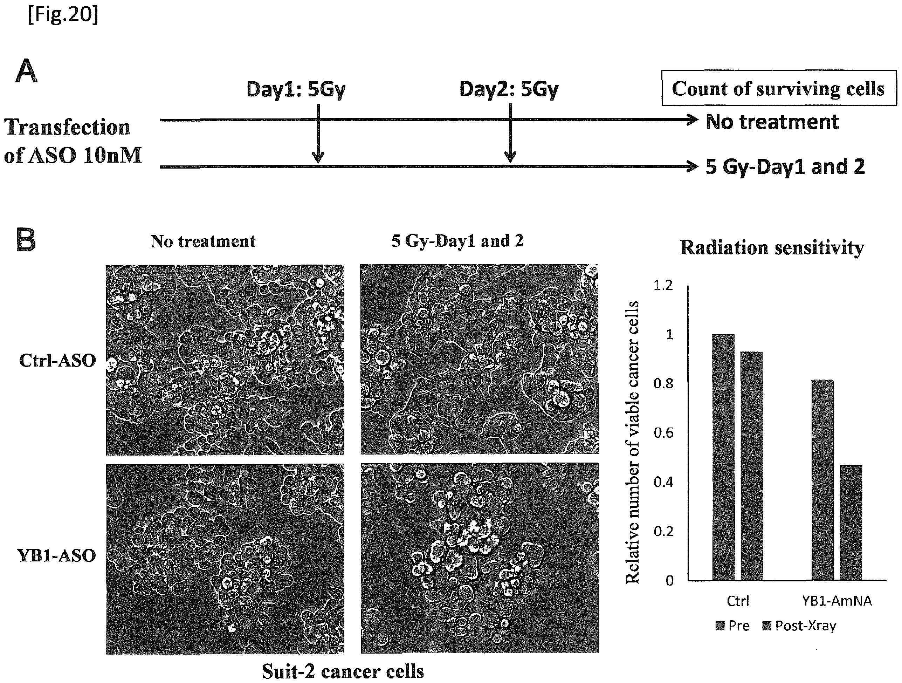

The #10 AmNAYB-1 antisense (10 nM) was introduced into cells of the pancreatic cancer cell strain SUIT-2 using the reagent Lipofectamine RNAiMax, and cell-killing effects of X-ray irradiation observed in the presence and absence of the antisense were compared. The results are shown in Fig. 20. Enhancement of the cell-killing effect by the introduction of the #10AmNA YB-1 antisense at a low concentration was observed.

### Example 21: Comparison of YB-1 knockdown efficiencies of antisenses prepared from naturally occurring nucleic acid, LNA/BNA, and AmNA nucleic acid

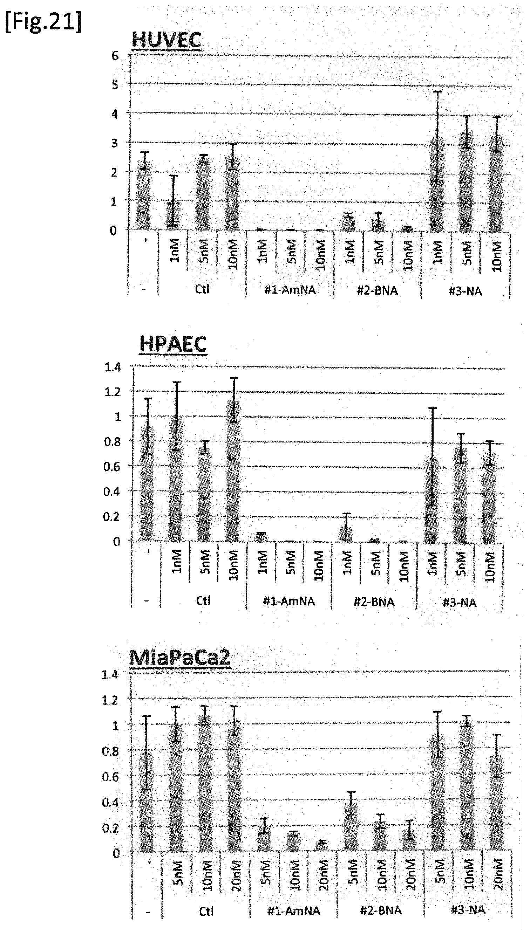

YB-1 naturally occurring type nucleic acid antisense (NA), LNA/BNA type nucleic acid antisense (BNA), and AmNA type nucleic acid antisense (AmNA) were introduced into cells of the vascular endothelial cell strains (HUVEC, HPAEC) and the pancreatic cancer cell strain (MiaPaCa2-) at various concentrations. RNAs were extracted 24 hours afterward, and the YB-1 expression was evaluated by real-time RT-PCR. The results are shown in Fig. 21. The YB-1 knockdown efficiency of the AmNA type nucleic acid antisense was significantly higher than those of the naturally occurring type nucleic acid antisense and LNA type nucleic acid antisense. The graphs show mean ± SD (n=3, *P<0.01 vs. BNA, P<0.0001 vs. NA)

### Example 22: Comparison of YB-1 knockdown efficiencies of siRNA and AmNA-AON

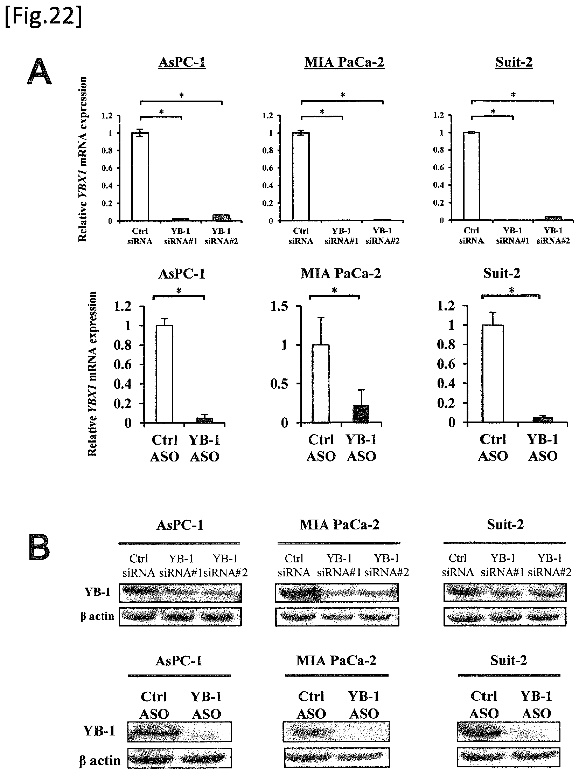

YB-1-siRNA or YB-1 # 10AmNA was introduced (final concentration, 20 nM) into cells of the pancreatic cancer cell strains (AsPC-1, MIA PaCa-2, and Suit-2). After 48 hours, RNAs and proteins were extracted, and the YB-1 expression was evaluated by real-time RT-PCR and Western blotting at the mRNA level (A) and protein level (B), respectively. The results are shown in Fig. 22. Both the knockdown efficiencies obtained with siRNA and #10 AmNA-AON were high at the mRNA level, and any clear difference was not observed between them. On the other hand, the knockdown efficiency of AmNA-AON at the protein level was clearly higher than that of siRNA. The graphs show mean ± SD (n=3, *P<0.05).

### Example 23: Comparison of YB-1 knockdown efficiencies of 15- to 18-mers

The 15- to 18-mer oligonucleotides were introduced into cells of the pancreatic cancer cell strain (MIA PaCa-2), large intestine cancer cell strain (HCT-116), and ovarian cancer cell strain (SKOV3) at various concentrations. After 24 hours, RNAs were extracted, and the YB-1 expression was evaluated by real-time RT-PCR. The structures of the used oligonucleotides other than the 15-mer are shown below.

[Formula 12] HsYB1-840-AmNA16 T (A) ^{^}T (A) ^{^5}(A) ^{^}t^{^}c^{^}c^{^}t^{^}g^{^}c^{^}a^{^}c^{^}c^{^5}(A) ^{^}T(A) ^{^}G(A) ^{^}g HsYB1-840-AmNA17 G(A) ^{^}T(A) ^{^}T(A) ^{^}c^{^}t^{^}c^{^}c^{^}t^{^}g^{^}c^{^}a^{^}c^{^}c^{^5}(A) ^{^}T(A) ^{^}G(A) ^{^}g HsYB1-840-AmNA18 T(A) ^{^}G(A) ^{^}T(A) ^{^}t^{^}c^{^}t^{^}c^{^}c^{^}t^{^}g^{^}c^{^}a^{^}c^{^}c^{^5}(A) ^{^}T(A) ^{^}G(A) ^{^}g HsYB1-839-AmNA16 T(A) ^{^5}(A) ^{^}T(A) ^{^}c^{^}c^{^}t^{^}g^{^}c^{^}a^{^}c^{^}c^{^}c^{^}T(A) ^{^}G(A) ^{^}G(A) ^{^}t HsYB1-839-AmNA17 T (A) ^{^}T(A) ^{^5}(A) ^{^}t^{^}c^{^}c^{^}t^{^}g^{^}c^{^}a^{^}c^{^}c^{^}c^{^}T(A) ^{^}G(A) ^{^}G(A) ^{^}t HsYB1-839-AmNA18 G(A) ^{^}T(A) ^{^}T(A) ^{^}c^{^}t^{^}c^{^}c^{^}t^{^}g^{^}c^{^}a^{^}c^{^}c^{^}c^{^}T(A) ^{^}G(A) ^{^}G(A) ^{^}t

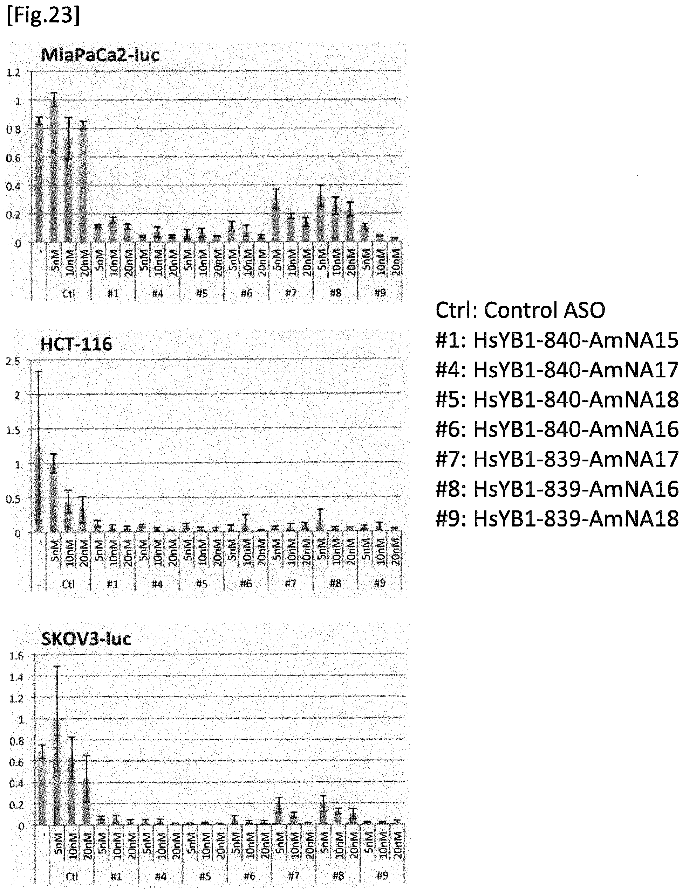

The results are shown in Fig. 23. For certain types of the cells, a higher YB-1 knockdown efficiency was provided by the 16-mer or 17-mer antisenses.

### Industrial Applicability

The present invention is useful in the fields of drug manufacturing, medical science, life science, and so forth.

## Claims

1. An oligonucleotide having a 13- to 17-nucleotide length, which comprises a part of the sequence of SEQ ID NO: 1 or 2 having at least 8-nucleotide length, and regulates expression of YB-1.

2. The oligonucleotide according to claim 1, which has a 14- to 16-nucleotide length.

3. The oligonucleotide according to claim 2, which has 15-nucleotide length.

4. An oligonucleotide consisting of the sequence of SEQ ID NO: 1 or 2.

5. The oligonucleotide according to any one of claims 1 to 4, which comprises at least one bridged nucleotide.

6. The oligonucleotide according to claim 5, wherein the bridged nucleotide has a nucleoside structure selected from the group consisting of the following nucleoside structures: 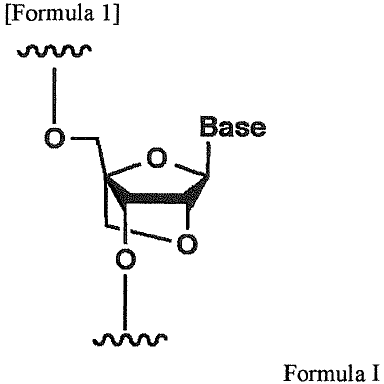 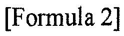 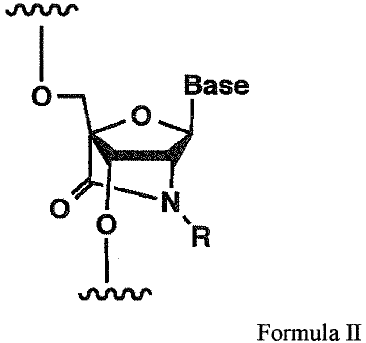 (in the formulas I and II:
Base represents a purin-9-yl group or 2-oxo-1,2-dihydropyrimidin-1-yl group which may have one or more arbitrary substituents selected from group α, wherein the group α consists of hydroxyl group, a hydroxyl group protected with a protective group for nucleic acid synthesis, a linear alkyl group having 1 to 6 carbon atoms, a linear alkoxy group having 1 to 6 carbon atoms, mercapto group, a mercapto group protected with a protective group for nucleic acid synthesis, a linear alkylthio group having 1 to 6 carbon atoms, amino group, a linear alkylamino groups having 1 to 6 carbon atoms, an amino group protected with one or more protective groups for nucleic acid synthesis, and a halogen atom; and
R represents hydrogen atom, an alkyl group having 1 to 7 carbon atoms, which may be branched or form a cyclic group, an alkenyl group having 2 to 7 carbon atoms, which may be branched or form a cyclic group, an aryl group having 3 to 12 carbon atoms, which may have one or more substituents selected from the group α, and may contain a heteroatom, an aralkyl group including an aryl moiety having 3 to 12 carbon atoms, which may have one or more substituents selected from the group α, and may contain a heteroatom, or a protective group for amino group used in nucleic acid synthesis.

7. The oligonucleotide according to any one of claims 1 to 6, which contains at least one phosphorothioate type nucleotide.

8. A method for suppressing expression of YB-1 in a cell or tissue, which comprises the step of contacting the oligonucleotide according to any one of claims 1 to 7 with the cell or tissue.

9. A method for treating a disease or condition relevant to expression of YB-1, which comprises the step of administrating the oligonucleotide according to any one of claims 1 to 7 to an object.

10. The method according to claim 9, wherein the disease or condition relevant to expression of YB-1 is a cancer.

11. The method according to claim 10, wherein the cancer is gastric cancer, large intestine cancer, esophageal cancer, breast cancer, pancreatic cancer, biliary tract cancer, lung cancer, malignant mesothelioma, or ovarian cancer.

12. The method according to claim 10 or 11, wherein the cancer is an anticancer agent-resistant cancer.

13. The method according to claim 12, wherein the anticancer agent-resistant cancer is gemcitabine-resistant pancreatic cancer.

14. A pharmaceutical composition containing the oligonucleotide according to any one of claims 1 to 7, and a pharmaceutically acceptable carrier.

15. The pharmaceutical composition according to claim 14, which is for treating a disease or condition relevant to expression of YB-1.

16. The pharmaceutical composition according to claim 15, which is for treating a cancer.

17. The pharmaceutical composition according to claim 16, which is for treating gastric cancer, large intestine cancer, esophageal cancer, breast cancer, pancreatic cancer, biliary tract cancer, lung cancer, malignant mesothelioma, or ovarian cancer.

18. The pharmaceutical composition according to claim 15 or 16, which is for treating an anticancer agent-resistant cancer.

19. The pharmaceutical composition according to claim 18, which is for treating gemcitabine-resistant pancreatic cancer.

20. The pharmaceutical composition according to any one of claims 14 to 19, which is for subcutaneous administration or oral administration.
